# EUROPÄISCHE PATENTANMELDUNG

(11) **EP 3 176 161 A1**
(43) Veröffentlichungstag der Anmeldung: **07.06.2017**
(21) Anmeldenummer: 15197483.9
(22) Anmeldetag: 02.12.2015
(51) Int. Cl.: C07D 403/12, C07F 15/00, C07F 17/02, C07D 209/86

(54) **BUCHWALD-HARTWIG ARYLIERUNGSVERFAHREN ZUR HERSTELLUNG TERTIÄRER ARYLAMINE**

(71) Anmelder: Umicore AG & Co. KG, 63457 Hanau-Wolfgang (DE)
(72) Erfinder: GOOSSEN, Lukas J., 67663 Kaiserslautern (DE); GRUENBERG, Matthias, 42285 Wuppertal (DE)

(57) **Zusammenfassung**

Die vorliegende Erfindung betrifft ein selektives Verfahren zur Herstellung eines tertiären Triaryl-Amins A-N(B)(C) unter Bedingungen der Hartwig-Buchwald-Kupplung. Bevorzugt ist einer der Arylreste ein Biphenylderivat.
Charakterisierend für das beanspruchte 2-stufige Verfahren ist, das die beiden Schritte durch einen Palladiumkomplex katalysiert werden, ausgewählt aus der Gruppe bestehend aus Bis-(Dialkylphosphinoferrocen)palladium-Komplexen oder Komplexen der Formel PdX₁X₂L₁L₂ wobei Xi und X₂ gleiche oder verschiedene Halogenliganden, Li und L₂ gleiche oder verschiedene neutrale Elektronendonorliganden sind, oder deren Kombinationen. Bevorzugt sind Pd-Katalysatoren der Formel PdX₁X₂L₁L₂ wobei Palladium-NHC Komplese (N-heterocylische Carben Komplexe).
Exemplarisch ein Beispiel des Verfahrens:

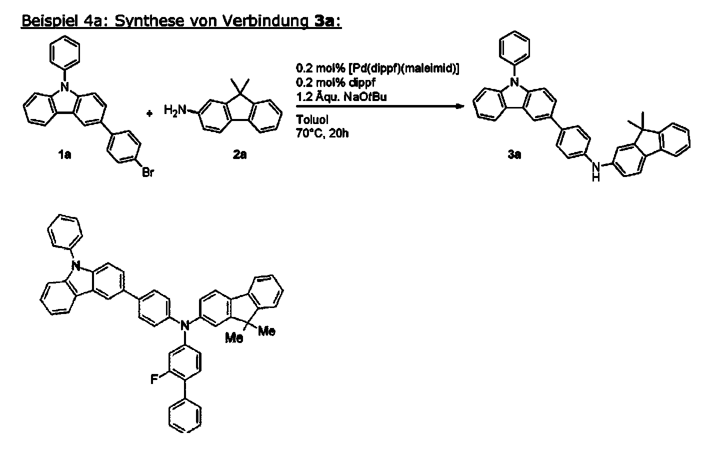

## Beschreibung

Die Hartwig-Buchwald-Reaktion hat mittlerweile Eingang in industrielle Fertigungsprozesse gefunden, insbesondere von Zwischenprodukten zur Herstellung organischer Leuchtdioden.
EP-A1-2407502 beschreibt die Herstellung von Dendrimeren für diesen Zweck, wobei sekundäre Amine als Zwischenprodukte durch die Hartwig-Buchwald-Reaktion erhalten werden.
Auch EP-A2-2421064 bedient sich der Hartwig-Buchwald-Reaktion als Syntheseweg für sekundäre Amine, die als Zwischenprodukte genutzt werden.

Die mäßige Ausbeute ist zwar nachteilig, das größere Problem stellt jedoch die mangelnde Selektivität dar, welche nicht nur zu sekundären, sondern auch tertiären Aminen als Nebenprodukte führt. Diese können nur schwer mittels Sublimation abgetrennt werden. Besonders nachteilig ist dabei außerdem, dass an Zwischenprodukte zur Herstellung organischer Leuchtdioden besonders hohe Reinheitsanforderungen bestehen, da unerwünschte, auch strukturverwandte Verunreinigungen, selbst in geringen Mengen zu einer unerwünschten Verschiebung der Emissionswellenlängen oder Verringerung der Quantenausbeute und damit auch zu unerwünschter Abwärmeentwicklung führen können.

Es ist die Aufgabe der vorliegenden Patentanmeldung, ein Verfahren bereit zu stellen, welches die Herstellung von Tribisphenylaminen unter den Bedingungen der Hartwig-Buchwald-Kupplung in höheren Ausbeuten und Selektivitäten ermöglicht, so dass der Aufwand für die Reinigung der Verbindungen, die meist durch Sublimation erfolgt, verringert werden kann. In Berücksichtigung des oben Erklärten ist es also erforderlich, die Ausbeuteerhöhung vor allem durch Erhöhung der Selektivität zu erreichen. Die Aufgabe wird gelöst durch ein Verfahren zur selektiven Arylierung gemäß der Patentansprüche und der weiteren Beschreibung.

### Kurze Beschreibung der Erfindung

Die Erfindung läßt sich in den folgenden Punkten kurz beschreiben:
1. Verfahren zur selektiven Arylierung eines primären aromatischen Amins der Formel A-NH₂ zu einem tertiären aromatischen Amin N-ABC, wobei die Reste A, B und C unabhängig voneinander gleiche oder verschiedene, substituierte oder unsubstituierte aromatische Reste sind, mindestens einer der Reste A, B oder C eine Biphenyleinheit aufweist, mit den Schritten
   - Umsetzung des primären aromatischen Amins der Formel A-NH₂ mit einer aromatischen Verbindung der Formel X-B, wobei X ein Halogen oder ein Trifluormethylsulfonsäurerest ist, in Gegenwart eines ersten Katalysators bei einer ersten Reaktionstemperatur, um ein sekundäres Amin zu erhalten;
   - Umsetzung des sekundären Amins mit einer aromatischen Verbindung der Formel X-C, wobei X ein Halogen oder ein Trifluormethylsulfonsäurerest ist, in Gegenwart eines zweiten Katalysators bei einer zweiten Reaktionstemperatur, um das tertiäre aromatische Amin N-ABC zu erhalten,
   wobei die zweite Reaktionstemperatur höher ist als die erste Reaktionstemperatur, die Reaktion in Gegenwart einer Base durchgeführt wird und der erste und der zweite Katalysator gleich oder verschieden sind und jeweils ein Palladiumkomplex sind, ausgewählt aus der Gruppe bestehend aus Bis-(Dialkylphosphinoferrocen)palladium-Katalysatoren, Katalysatoren der Formel PdX₁X₂L₁L₂ wobei X₁ und X₂ gleiche oder verschiedene Halogenliganden, L₁ und L₂ gleiche oder verschiedene neutrale Elektronendonorliganden sind, oder deren Kombinationen.
2. Verfahren nach Punkt 1, wobei X1 und X2 gleich und ausgewählt aus F, Cl, Br oder I sind.
3. Verfahren nach Punkt 1 oder 2, wobei L1 und L2 verschieden sind.
4. Verfahren nach einem oder mehreren der Punkte 1 bis 3, wobei L1 und L2 substituierte oder unsubstituierte neutrale aromatische oder heteroaromatische Verbindungen sind.
5. Verfahren nach einem oder mehreren der Punkte 1 bis 4, wobei L1 ein NHC-Ligand ist.
6. Verfahren nach einem oder mehreren der Punkte 1 bis 5, wobei L2 ein Pyridylligand ist.
7. Verfahren nach einem oder mehreren der Punkte 1 bis 6, wobei L1 ein NHC-Ligand ausgewählt aus der Gruppe bestehend aus 1,3-Bis-(2,4,6-Trimethylphenyl)-imidazolidin-2-yliden ("SIMes"), 1,3-Bis-(2,6-Di-isopropylphenyl)-imidazolidin-2-yliden ("SIPr") oder 1,3-Bis-(2,6-Di-isopropylphenyl)-imidazolin-2-yliden (ungesättigter NHC, "IPr"), Bis-(2,6-Di-(1-Propylbutylphenyl))-4,5-Dichlorimidazolin-2-yliden und 1,3-Bis-(2,6-Di-(1-Ethylpropylphenyl))-4,5-Dichlorimidazolin-2-yliden ist und L2 ein Pyridin, Pyrimidin oder Pyrazin, welches optional ein- oder zweifach mit Methyl, Ethyl, Propyl, Isopropyl, tert.-Butyl, Chlor, Brom oder Iod oder deren Kombinationen substituiert ist.
8. Verfahren nach einem oder mehreren der Punkte 1 bis 7, wobei die Alkylsubstituenten der Bis-(Dialkylphosphinoferrocen)-Liganden zwei bis fünf Kohlenstoffatome aufweisen.
9. Verfahren nach einem oder mehreren der Punkte 1 bis 8, wobei in dem sekundären aromatischen Amin die aromatischen Kohlenstoffatome direkt an das Stickstoffatom gebunden sind, mindestens einer der Reste A oder B eine Biphenyleinheit aufweist und die Reaktion in Gegenwart einer Base und eines Palladiumkomplexes durchgeführt wird, wobei das Palladiumatom von mindestens einem Bis-(Dialkylphosphinoferrocen)-Liganden komplexiert wird.
10. Verfahren nach einem oder mehreren der Punkte 1 bis 9, wobei die Alkylsubstituenten der Bis-(Dialkylphosphinoferrocen)-Liganden zwei bis fünf Kohlenstoffatome aufweisen.
11. Verfahren nach einem oder mehreren der Punkte 1 bis 10, wobei die Alkylsubstituenten ausgewählt sind aus der Gruppe bestehend aus Isopropyl, Isobutyl, tert.-Butyl und deren Kombinationen.
12. Verfahren nach einem der Punkte 1 bis 11, wobei mindestens zwei der aromatischen Reste A, B oder C eine Biphenyleinheit aufweisen, welche gleich oder verschieden voneinander sind.
13. Verfahren nach einem der Punkte 1 bis 12, wobei die Biphenyleinheit direkt an das sekundäre Stickstoffatom des Amins gebunden ist.
14. Verfahren nach einem der vorstehenden Punkte, wobei die Alkylsubstituenten ausgewählt sind aus der Gruppe bestehend aus Isopropyl, Isobutyl und deren Kombinationen.
15. Verfahren nach einem der vorstehenden Punkte, wobei die Biphenyleinheit direkt an die Abgangsgruppe, insbesondere ein Halogen, also Chlor, Brom, Iod oder an eine Trifluormethylsulfonsäuregruppe gebunden ist.
16. Verfahren nach einem der vorstehenden Punkte, wobei die Biphenyleinheit eine verbrückte Biphenyleinheit der Formel 2 oder 3 ist 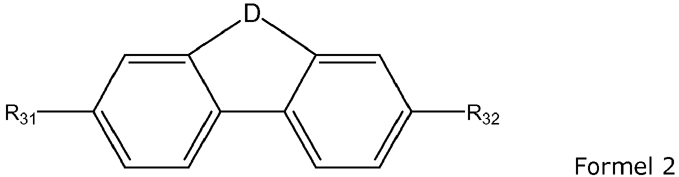 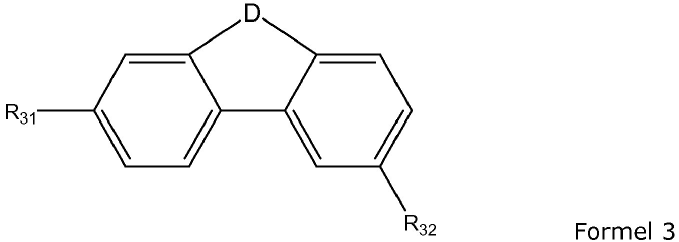 wobei D Sauerstoff, Schwefel, Stickstoff oder Kohlenstoff sein und im Fall von Stickstoff einfach oder im Fall von Kohlenstoff zweifach mit Methyl, Ethyl, Biphenyl, Naphthyl oder Phenyl substituiert sein kann und wobei R₃₁ Wasserstoff, Phenyl, Biphenyl oder Pyridyl sein kann, R₃₂ eine Abgangsgruppe, Halogen, eine primäre Amingruppe NH2 oder ein Trifluormethylsulfonsäurerest sein kann, je nachdem ob der Rest als A, B oder C verwendet wird; oder aber R₃₂ ein Spacer ist, welcher zwischen A, B oder C und X bzw. NH₂ angeordnet ist.
17. Verfahren nach einem oder mehreren der vorstehenden Punkte, wobei mindestens einer der aromatischer Reste A, B, C oder mehrere gleich oder verschieden und ausgewählt sind aus einer Einheit der Formeln 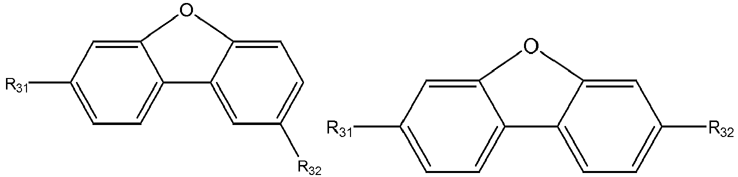 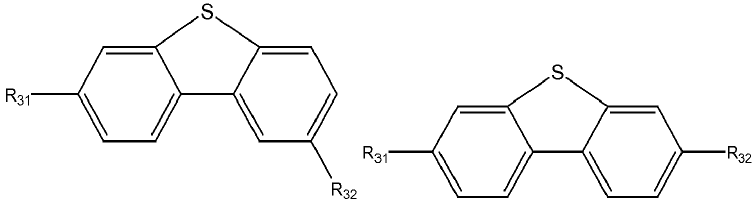 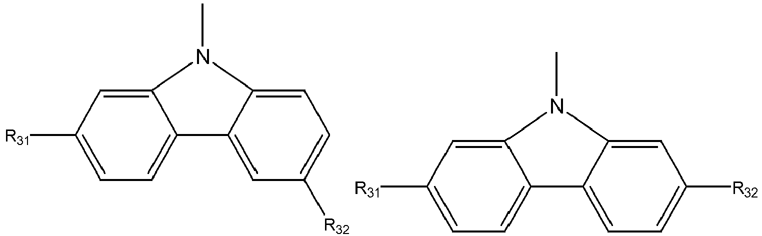 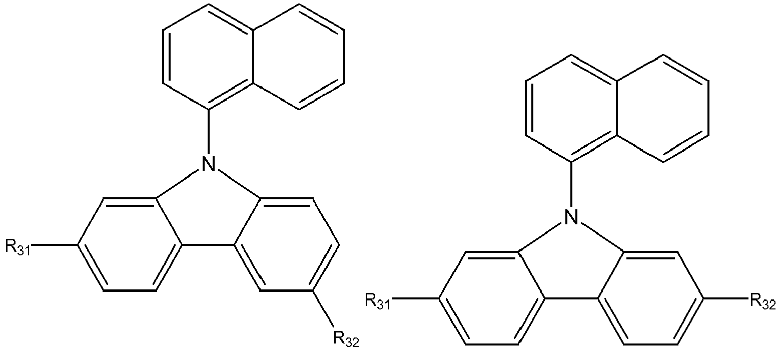 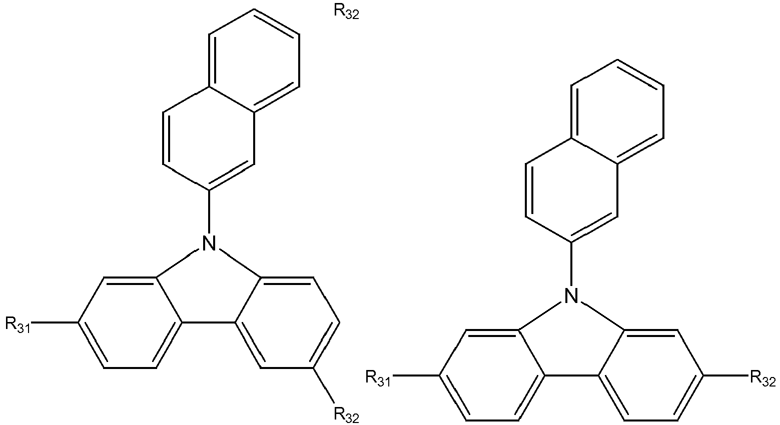 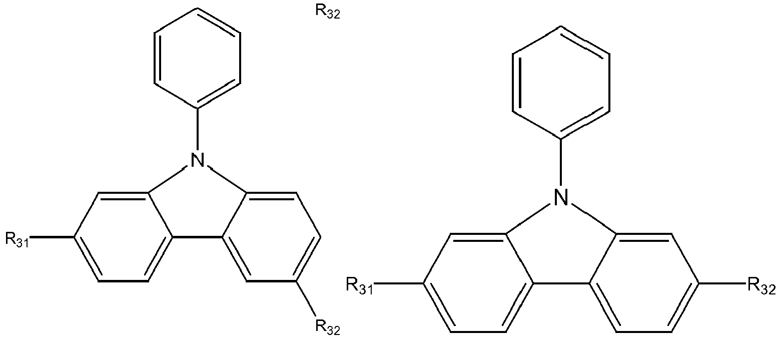 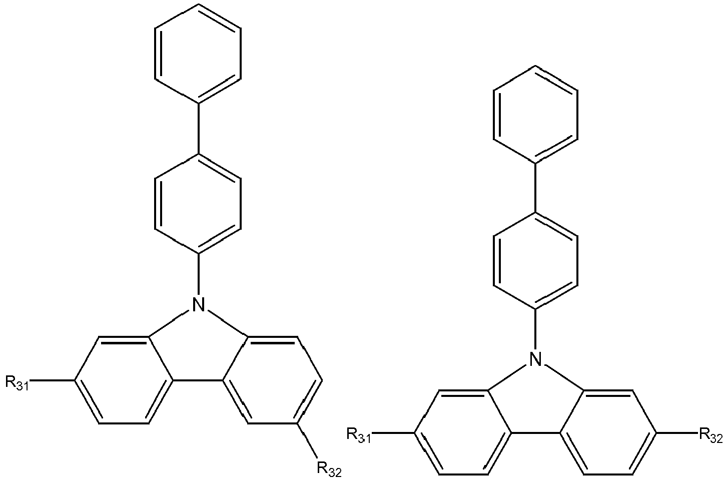 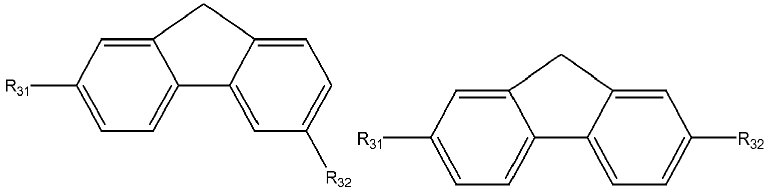 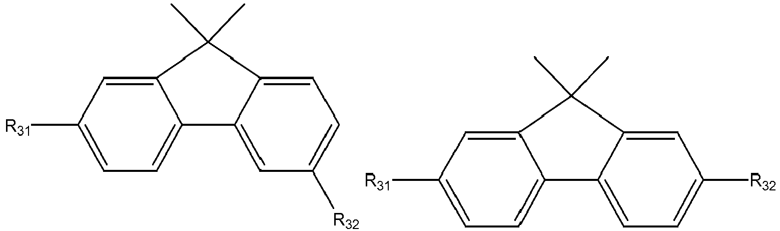 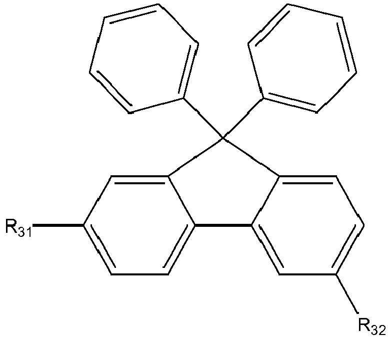 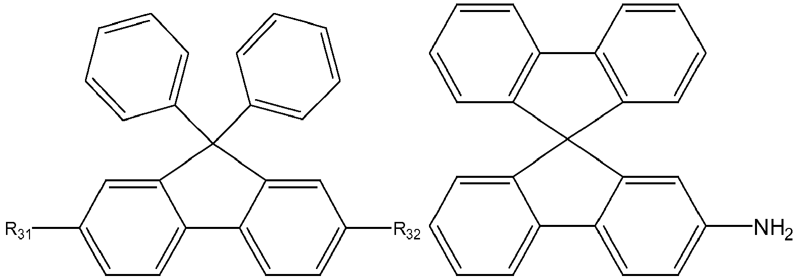 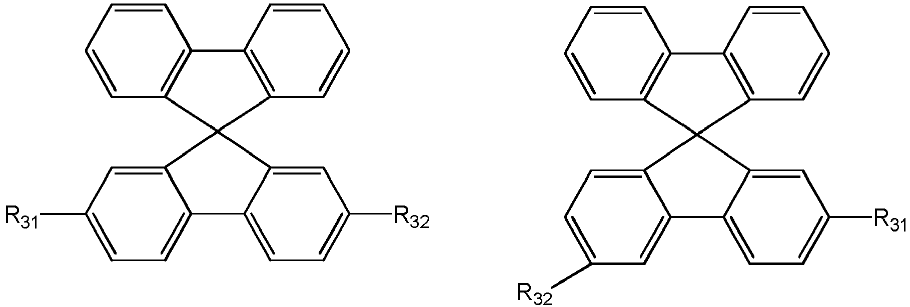 wobei R31 Wasserstoff, Phenyl, Biphenyl oder Pyridyl sein kann, R32 eine Abgangsgruppe, Halogen, eine primäre Amingruppe NH₂ oder ein Trifluormethylsulfonsäurerest sein kann, je nachdem ob der Rest als A, B oder C verwendet wird; oder aber R32 ein Spacer ist, welcher zwischen A bzw. B oder C und X bzw. NH₂ angeordnet ist.
18. Verfahren nach einem oder mehreren der vorstehenden Punkte, wobei der Spacer ausgewählt ist aus der Gruppe bestehend aus 1,4-Phenyl, 1,4-(6-Methyl)phenyl, 1,4-(5-Methyl)phenyl, 4,4'-Biphenyl, 2,6-Naphthyl, 1,4-Naphthyl, 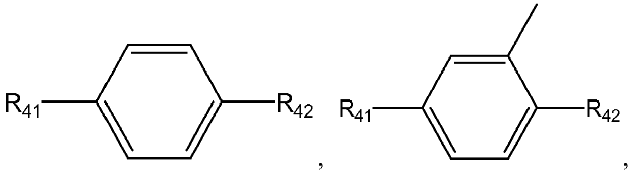 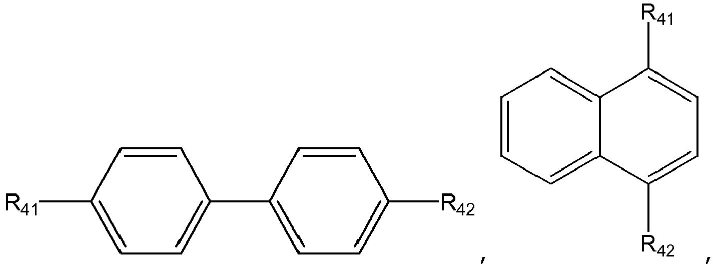 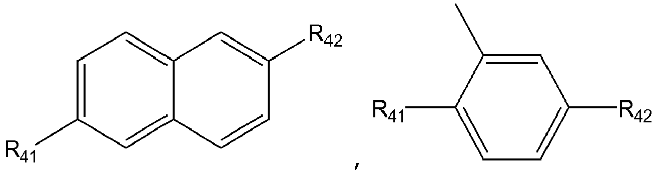 und wobei R41 hierbei eine Struktur der Formeln 2 bis 22 ist, R42 Halogen wie Fluor, Chlor, Brom, Iod oder Astat, eine primäre Amingruppe NH₂ oder ein Trifluormethylsulfonsäurerest ist.
19. Verfahren nach einem oder mehreren der vorstehenden Punkte, wobei die Base Lithiumhydroxid, Kaliumhydroxid, Natriumhydroxid, tertiäre organische Amine, Alkoholate, Tributylamin, Triethylamin, Alkalimetallalkoholate, Lithiumethanolat, Natriumethanolat, Kaliumethanolat, Lithium-tert.-butanolat, Natrium-tert.-butanolat, Kalium-tert.-butanolat, Lithiumbis(trimethylsilyl)amid, Natriumbis(trimethylsilyl)amid, Kaliumbis(trimethylsilyl)amid, Natrium- oder Kaliumsalze von BHT (2,6-Di-tert.-butylhydroxytoluol), oder deren Kombinationen ist.
20. Verfahren nach einem oder mehreren der vorstehenden Punkte, wobei das Verfahren in einem Lösungsmittel ausgewählt aus der Gruppe bestehend aus Alkoholen, Ethanol, Propanol, Isopropanol, Butanol, Isobutanol, Ethylenglykol, Ether, Diethylether, tert.-Butylethylether, tert.-Butylmethylether, Tetrahydrofuran, Dioxan Ethylenglykoldimethylether, Bis(2-methoxyethyl)ether, aromatische Lösungsmittel, Benzol, Toluol, Xylol, o-Xylol, p-Xylol, m-Xylol und deren Kombinationen durchgeführt wird.
21. Verfahren nach einem oder mehreren der vorstehenden Punkte, wobei die erste Temperatur 5°C bis 95°C, 30°C bis 90°C, 50°C bis 80°C oder 55°C bis 70°C, die zweite Temperatur von 90°C bis 144°C, 100°C bis 139°C, 111°C bis 138°C oder 115°C bis 125°C beträgt.
22. Verfahren nach einem oder mehreren der vorstehenden Punkte, wobei die Reaktionszeit des Verfahrensschrittes zur Herstellung des sekundären und des Verfahrensschrittes zur Herstellung des tertiären Amins jeweils von einer Stunde bis 36 Stunden, 4 Stunden bis 24 Stunden, 6 Stunden bis 16 Stunden oder 8 Stunden bis 12 Stunden liegt.
23. Verfahren nach einem oder mehreren der vorstehenden Punkte, wobei die Biphenyleinheit 2-Fluoren, 3-Fluoren, 2-(9,9-Diphenylfluoren), 2-(9,9-Dimethylfluoren), 3-(9,9-Diphenylfluoren), 3-(9,9-Dimethylfluoren), 3-(4-phenyl)-9-phenyl-9H-carbazol, 3-(4-phenyl)-9-methyl-9H-carbazol, 3-(4-phenyl)-9-Biphenyl-9H-carbazol, 2-(4-phenyl)-9-phenyl-9H-carbazol, 2-(4-phenyl)-9-methyl-9H-carbazol, 2-(4-phenyl)-9-Biphenyl-9H-carbazol, 3-(4-Biphenyl)-9-phenyl-9H-carbazol, 3-(4-Biphenyl)-9-methyl-9H-carbazol, 3-(4-Biphenyl)-9-Biphenyl-9H-carbazol, 2-(4-Biphenyl)-9-phenyl-9H-carbazol, 2-(4-Biphenyl)-9-methyl-9H-carbazol, 2-(4-Biphenyl)-9-Biphenyl-9H-carbazol, -- 3-(9-phenyl-9H-carbazol), 3-(9-methyl-9H-carbazol), 3-(9-Biphenyl-9H-carbazol), 2-(9-phenyl-9H-carbazol), 2-(9-methyl-9H-carbazol), 2-(9-Biphenyl-9H-carbazol) oder Triphenylene ist.
24. Verfahren nach einem oder mehreren der vorstehenden Punkte, wobei der Palladiumkomplex in Mengen von 0,01 mol% bis 1,5 mol%, bezogen auf die molare Gesamtmenge an beiden Edukten, dem aromatischen Amin A-NH₂ oder AB-NH und dem Halogenaromaten X-B oder X-C, eingesetzt wird.
25. Verfahren nach einem oder mehreren der vorstehenden Punkte, wobei der Palladiumkomplex als Feststoff, Lösung oder Pulvermischung mit einem festen Bis-(Dialkylphosphinoferrocen) verwendet wird.
26. Verfahren nach einem der vorstehenden Punkte enthaltend die Schritte:
   - Bereitstellen des primären aromatischen Amins A-NH₂, des Halogenaromaten X-B, eines geeigneten Lösemittels und gegebenenfalls eines Bis-(Dialkylphosphinoferrocens) in einem Reaktionsgefäß;
   - Zugeben eines Palladiumkomplexes, bei welchem das Palladiumatom von mindestens einem Bis-(Dialkylphosphinoferrocen)-Liganden komplexiert wird, in Form eines Feststoffs oder einer Lösung;
   - Erwärmen des so erhaltenen Reaktionsgemisches in dem Reaktionsgefäß;
   - Abtrennen des Reaktionsproduktes, eines sekundären aromatischen Amin A-NH-B; und
   - Gegebenenfalls Reinigung des sekundären aromatischen Amin A-NH-B.
27. Verfahren nach einem der vorstehenden Punkte enthaltend die Schritte:
   - Bereitstellen des primären aromatischen Amins A-NH₂, des Halogenaromaten X-B, eines geeigneten Lösemittels und gegebenenfalls eines Bis-(Dialkylphosphinoferrocens) in einem Reaktionsgefäß;
   - Zugeben eines Palladiumkomplexes, bei welchem das Palladiumatom von mindestens einem Bis-(Dialkylphosphinoferrocen)-Liganden komplexiert wird, in Form eines Feststoffs oder einer Lösung;
   - Erwärmen des so erhaltenen Reaktionsgemisches auf die erste Reaktionstemperatur;
   - Zugeben eines Halogenaromaten X-C;
   - Erwärmen auf die zweite Reaktionstemperatur; und
   - Abtrennung und gegebenenfalls Reinigung des tertiären aromatischen Amin N-ABC.
28. Verfahren nach einem oder mehreren der vorstehenden Punkte, wobei die Verbindungen 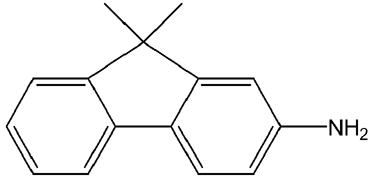 und 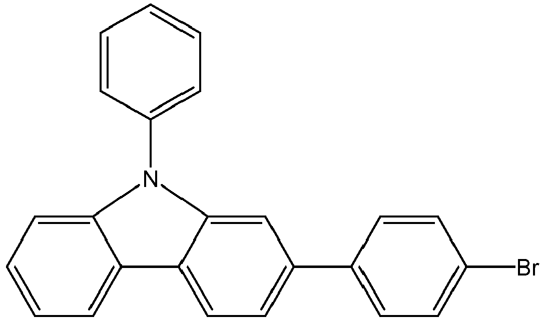 vorhanden sind.
29. Verfahren nach einem oder mehreren der vorstehenden Punkte, wobei in dem Palladiumkomplex das Palladiumatom zusätzlich von 2,4,6,8-Tetramethylcyclotetrasiloxan, Bis(dibenzylidenaceton) oder Maleimid komplexiert wird.
30. Verfahren nach einem oder mehreren der vorstehenden Punkte, wobei das Palladiumatom in dem Palladiumkomplex von 1,1'-Bis(diisopropylphosphino)ferrocen komplexiert wird.
31. Verfahren nach einem oder mehreren der vorstehenden Punkte, wobei in dem sekundären aromatischen Amin die aromatischen Kohlenstoffatome direkt an das Stickstoffatom gebunden sind.

### Detaillierte Beschreibung der Erfindung

Der erste Katalysator und der zweite Katalysator sind beide Palladiumkomplexe. Sie können gleich oder verschieden sein.

In dem Palladiumkomplex kann das Palladiumatom von mindestens einem Bis-(Dialkylphosphinoferrocen)-Liganden komplexiert sein, welcher die allgemeine Formel 1 aufweist:

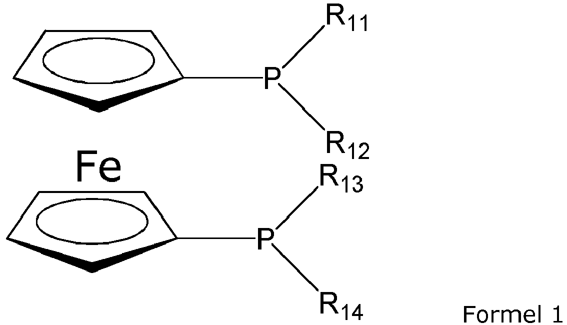

In diesem Fall spricht man von einem Bis-(Dialkylphosphinoferrocen)palladium-Komplex bzw. Bis-(Dialkylphosphinoferrocen)palladium-Katalysator.

Bei diesen Komplexen können R11 bis R14 gleich oder verschieden sein und sind insbesondere Alkylreste mit einem bis fünf Kohlenstoffatomen. R11 bis R14 können also unabhängig voneinander ausgewählt sein aus Methyl, Ethyl, n-Propyl, Isopropyl, n-Butyl, Isobutyl, sec.-butyl, tert.-Butyl, n-Pentyl (Amyl), 2-Pentyl (sec-Pentyl), 3-Pentyl, 2-Methylbutyl, 3-Methylbutyl (iso-Pentyl oder iso-Amyl), 3-Methylbut-2-yl, 2-Methylbut-2-yl,2,2-Dimethylpropyl (Neopentyl).

Vorteilhaft sind R11 bis R14 gleich und ausgewählt aus der Gruppe bestehend aus Methyl, Ethyl, n-Propyl, Isopropyl, n-Butyl, Isobutyl, sec.-butyl, tert.-Butyl, n-Pentyl (Amyl), 2-Pentyl (sec-Pentyl), 3-Pentyl, 2-Methylbutyl, 3-Methylbutyl (iso-Pentyl oder iso-Amyl), 3-Methylbut-2-yl, 2-Methylbut-2-yl,2,2-Dimethylpropyl (Neopentyl).

Besonders geeignet sind Isopropyl, Isobutyl, tert.-Butyl, insbesondere Isopropyl und tert.-Butyl. Gute Ergebnisse werden erzielt, wenn R11 bis R14 gleich und Isopropyl oder tert.-Butyl sind. Gute Ergebnisse werden insbesondere erzielt, wenn R11 bis R14 gleich und Isopropyl, Propyl oder Isobutyl sind. Gute Ergebnisse werden insbesondere erzielt, wenn R11 bis R14 gleich und Isopropyl sind.

Der Palladiumkomplex, der als erster oder zweiter Katalysator verwendet werden kann, kann auch ein Palladiumkomplex der allgemeinen Formel PdX₁X₂L₁L₂ sein, wobei X₁ und X₂ gleiche oder verschiedene Halogenliganden, L₁ und L₂ gleiche oder verschiedene neutrale Elektronendonorliganden sind. Als Halogenliganden X₁ und X₂ können im Allgemeinen Fluor, Chlor, Brom oder Iod eingesetzt werden, insbesondere Chlor oder Brom.

Als neutrale Elektronendonorliganden verwendet werden können beispielsweise Phosphine, wie Tri-*iso*-propylphosphin, Tricyclohexylphosphin (PC_{Y3}) oder Tricyclopentylphosphin, Triphenylphosphin, Triorthotolylphosphin. Ebenfalls geeignet sein können Phospha-bicycloalkane wie 9-Phosphabicyclo-[3.3.1]-nonan oder 9-Phosphabicyclo-[4.2.1]-nonan (auch als "phobane" bekannt), ebenfalls geeignet sind 9-Cyclohexyl-9-phospha-bicyclo-[3.3.1]-nonan ("cyclohexylphoban"), 9-(2,2,4-trimethylpentyl)-9-Phospha-bicyclo-[3.3.1]-nonan ("2,2,4-trimethylpentyl phoban") und 9-Isobutyl-9-phospha-bicyclo-[3.3.1]-nonan ("isobutylphobane").

Ebenfalls geeignet sind N-heterocyclische Carbenliganden, die auch als "NHC-Liganden" bezeichnet werden.

Gut geeignet sind hierbei stabile stickstoffhaltige Heterocyclen, die mindestens ein Stickstoffatom enthalten und mehrere Kohlenstoffatome als Ringatome aufweisen. Mindestens eines der Stickstoffatome im Ring ist mit einem organischen Rest verbunden, der nicht Teil der heterocyclischen Ringstruktur ist. Insbesondere geeignet sind NHC-Liganden der Formeln (III) und (IV), insbesondere der Formel (IV).

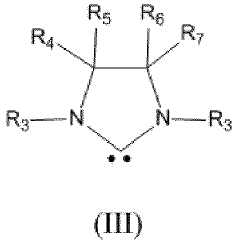

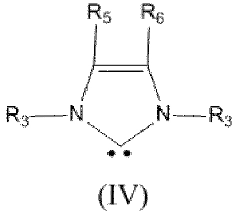

In den Formeln (III) und (IV) ist R₃ eine substitutierte Arylgruppe ausgewählt aus der Gruppe bestehend aus 2,4,6-Trimethylphenyl ("Mesityl"), 2,6-Di-*iso*propylphenyl, 2,6-Di-(1-Ethylpropyl)phenyl, 2,6-Di-(1-Propylbutyl)phenyl, 3,5-Di-*tert*.-butylphenyl und 2-Methylphenyl und deren Kombinationen. Gut geeignet ist 2,6-Di-(1-Ethylpropylphenyl), welches auch als 2,6-Di-(3-Pentyl)phenyl aufgefasst werden kann, oder 2,6-Di-(1-Propylbutyl)phenyl, welches auch als 2,6-Di-(4-Heptyl)phenyl oder auch aufgefasst werden kann.

In den Formeln (III) und (IV) sind R₅ und R₆ unabhängig voneinander ausgewählt aus Wasserstoff, Halogen, Methyl, Nitrogruppen oder R₅ und R₆ bilden einen Ring mit insgesamt 4 bis 8, insbesondere 5 bis 6 Kohlenstoffatomen. Insbesondere sind R₅ und R₆ unabhängig voneinander ausgewählt aus Wasserstoff, Chlor, Brom, Iod oder deren Kombinationen. In einer spezifischen Ausführungsform sind R₅ und R₆ gleich.

In den Formeln (III) und (IV) sind R₄ und R₇ unabhängig voneinander ausgewählt aus Wasserstoff, Halogen, Methyl, Nitrogruppen und deren Kombinationen. Insbesondere sind R₄ und R₇ gleich und ausgewählt aus Wasserstoff, Chlor, Brom oder Iod. Insbesondere sind R₄ und R₇ Wasserstoff.

Der NHC-Ligand ist insbesondere ausgewählt aus der Gruppe bestehend aus 1,3-Bis-(2,4,6-Trimethylphenyl)-imidazolidin-2-yliden ("SIMes"), 1,3-Bis-(2,6-Di-isopropylphenyl)-imidazolidin-2-yliden ("SIPr") oder 1,3-Bis-(2,6-Di-isopropylphenyl)-imidazolin-2-yliden (ungesättigter NHC, "IPr"), Bis-(2,6-Di-(1-Propylbutylphenyl))-4,5-Dichlorimidazolin-2-yliden oder 1,3-Bis-(2,6-Di-(1-Ethylpropylphenyl))-4,5-Dichlorimidazolin-2-yliden.
Der NHC-Ligand ist insbesondere als L₁ geeignet.

Als neutrale Elektronendonorliganden ebenfalls geeignet sind Pyridylliganden. Dies sind Pyridin, Pyrimidin, Pyrazin und deren Derivate, also substituiertes Pyridin, Pyrimidin oder Pyrazin. Die Komplexierung des Palladiumatoms findet hierbei über das heterocyclische Stickstoffatom statt. Pyridin, Pyrimidin oder Pyrazin können mit Alkyl, Nitro, Halogen ein- oder zweifach substituiert sein, insbesondere Methyl, Ethyl, Propyl, Isopropyl, tert.-Butyl, Chlor, Brom oder Iod. In einer speziellen Ausführungsform sind Pyridin, Pyrimidin oder Pyrazin einfach substituiert mit Methyl, Ethyl, Propyl, Isopropyl, tert.-Butyl, Nitro, Chlor, Brom oder Iod. Alkylsubstituenten befinden sich hierbei insbesondere in 2- bzw. 6-("ortho") oder 4- ("para") Stellung zum komplexierenden Stickstoffatom, also dem Stickstoffatom, welches das Palladium koordiniert. Nitrogruppen oder Halogenatome (Fluor, Chlor, Brom, Iod) sind insbesondere in 3-Stellung ("meta"-Stellung) zum komplexierenden Stickstoffatom angeordnet.

Geeignet sind daher insbesondere Pyridin, Pyrimidin, Pyrazin, 3-Chlorpyridin, 4-Methylpyridin, 2-Methylpyridin, 2,6-Dimethylpyridin, 5-Chlorpyrimidin, 4-Methylpyrimidin, 3-Chlorpyrazin.

Pyridin, Pyrimidin, Pyrazin und deren Derivate sind insbesondere als L₂ gut geeignet.

In einer spezifischen Ausgestaltung sind im Palladiumkomplex PdX₁X₂L₁L₂ die Halogenliganden X₁ und X₂ gleich und Chlor oder Brom, L₁ und L₂ voneinander verschieden, wobei L₁ ein NHC-Ligand ist und L₂ ein Pyridin, Pyrimidin, Pyrazin oder eines deren Derivate ist.

Insbesondere sind die Halogenliganden X₁ und X₂ gleich und Chlor, L₁ ist ein NHC-Ligand ist insbesondere ausgewählt aus der Gruppe bestehend aus 1,3-Bis-(2,4,6-Trimethylphenyl)-imidazolidin-2-yliden ("SIMes"), 1,3-Bis-(2,6-Diisopropylphenyl)-imidazolidin-2-yliden ("SIPr") oder 1,3-Bis-(2,6-Diisopropylphenyl)-imidazolin-2-yliden (ungesättigter NHC, "IPr"), oder 1,3-Bis-(2,6-Di-(1-Ethylpropylphenyl))-4,5-Dichlorimidazolin-2-yliden, Bis-(2,6-Di-(1-Propylbutylphenyl))-4,5-Dichlorimidazolin-2-yliden und L₂ ist ausgewählt aus der Gruppe bestehend aus Pyridin, Pyrimidin, Pyrazin, 3-Chlorpyridin, 4-Methylpyridin, 2-Methylpyridin, 2,6-Dimethylpyridin, 5-Chlorpyrimidin, 4-Methylpyrimidin, 3-Chlorpyrazin.

Spezifische geeignete Verbindungen sind die als Pd-PEPPSI^{pyr}-IPR, Pd-PEPPSI^{2,6-Di-Me}-IPR, Pd-PEPPSI^{2Me}-IPR, Pd-PEPPSI^{pyr}-IPR, Pd-PEPPSI²_{'}^{6-Di-Me}-IPR^{Cl}, Pd-PEPPSI^{pyr}-IPent, Pd-PEPPSI^{2Me}-IPent, Pd-PEPPSI-IPR^{Me}, Pd-PEPPSI-IPR^{Cl}, Pd-PEPPSI-IPR^{Qino}, Pd-PEPPSI-IHEPT, Pd-PEPPSI-IHEPT^{Cl} kommerziell verfügbare Verbindungen der folgenden Formeln.

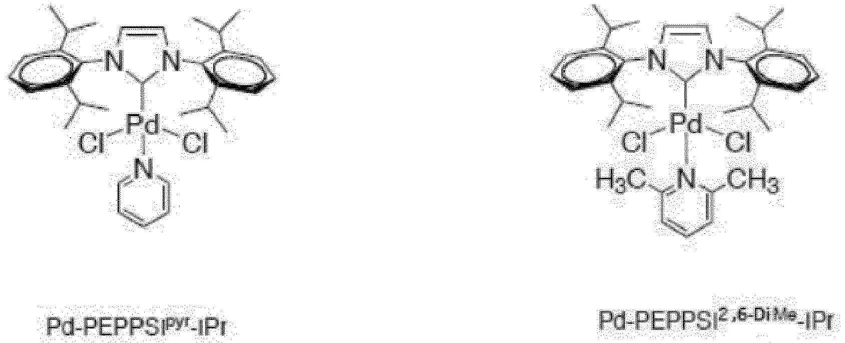

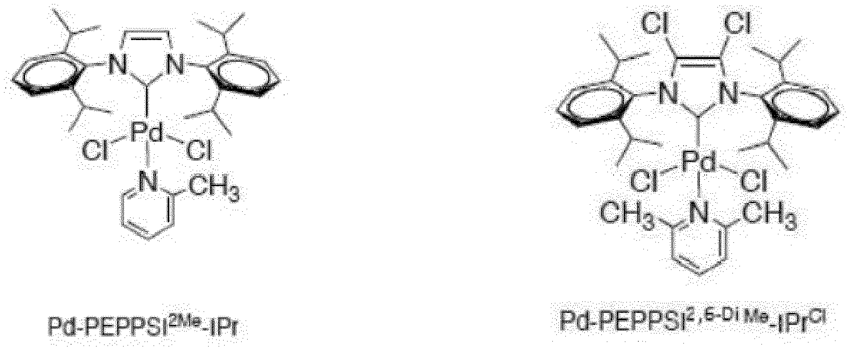

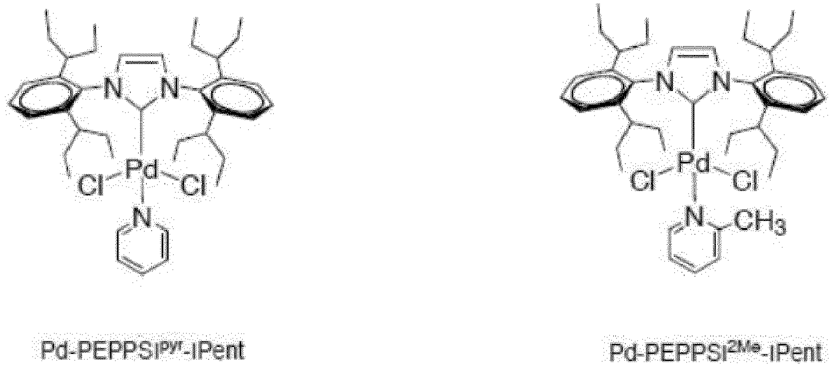

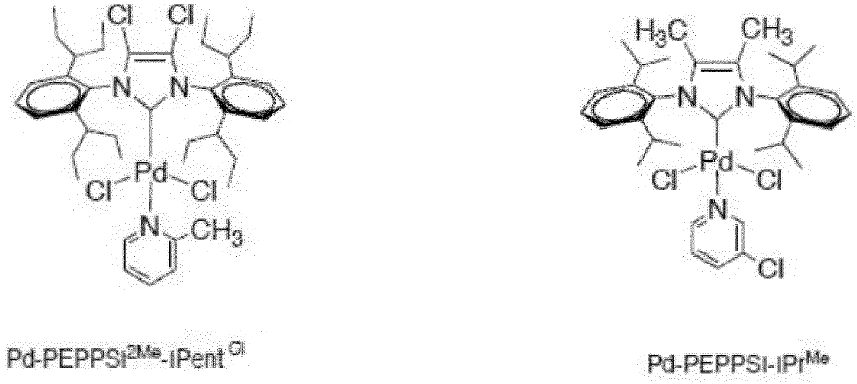

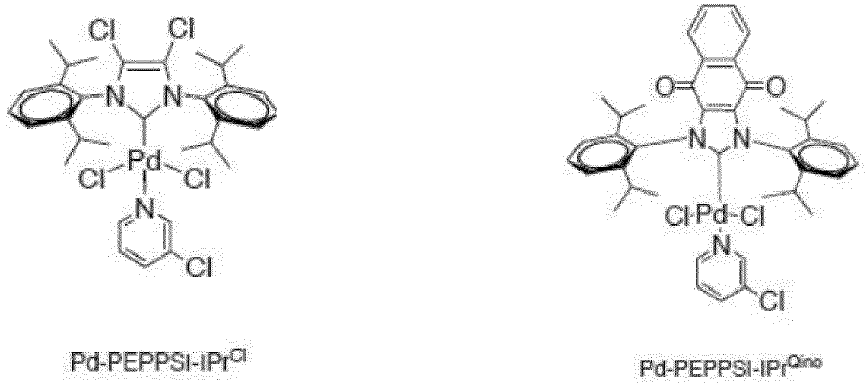

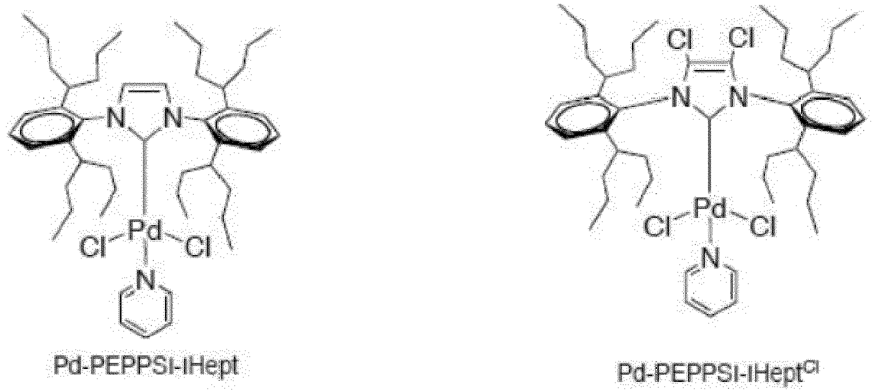

Gut geeignet ist insbesondere ein Palladiumkomplex PdX₁X₂L₁L₂, bei welchem die Halogenliganden X₁ und X₂ Chlor, L₁ Bis-(2,6-Di-(1-Ethylpropylphenyl))-4,5-Dichlorimidazolin-2-yliden und L₂ 3-Chlorpyridin ist. Dieser Komplex besitzt die Struktur von Formel (V) und ist kommerziell erhältlich, beispielsweise unter der Bezeichnung Pd-PEPPSI-IPent^{Cl} (Total Synthesis Ltd.).

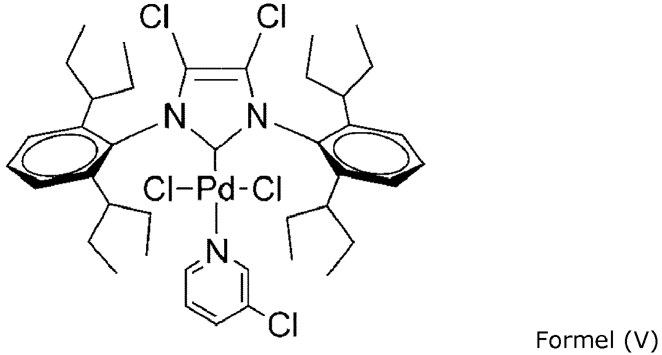

Es wurde überraschend gefunden, dass Palladiumkomplexe des Bis(Dialkylphosphinoferrocen)-Liganden der Formel 1 nicht nur bei der Herstellung von sekundären Aminen durch Hartwig-Buchwald-Kupplung die selektive Herstellung der sekundären Amine mit hohen Ausbeuten gestatten, wenn zumindest einer der Reste A oder B der zu kuppelnden Verbindungen als Strukturelement mindestens eine Biphenyleinheit aufweist, sondern bei erhöhter Temperatur auch zur Weiterreaktion zum tertiären Amin durch Reaktion des so erhaltenen sekundären Amins mit einer Verbindung des Typs X-C gut geeignet ist.

Gleiches gilt für Palladiumkomplexe der allgemeinen Formel PdX₁X₂L₁L₂, die ebenfalls oben beschrieben sind.

Hierbei kann die verwendete Palladiummenge von den normalerweise verwendeten 3 mol% auf etwa 1,5 mol% oder weniger gesenkt werden, wobei die normalerweise als Nebenreaktion auftretende Doppelarylierung zum tertiären Amin unterdrückt wird und die Reaktion zum gewünschten Produkt mit Umsätzen von meist 90% oder mehr abläuft. Gleiches wurde überraschend für Palladiumkomplexe der allgemeinen Formel PdX₁X₂L₁L₂, die oben beschrieben sind, insbesondere für die von TotalSynthesis Ltd. vermarkteten Palladiumkatalysatoren mit der Bezeichnung "Pd-PEPPSI" beobachtet.

Vorzugsweise weisen mindestens zwei der Reste A, B und C eine Biphenyleinheit auf. Die Biphenyleinheit kann unsubstituiert oder substituiert sein, auch mit einem oder mehreren Phenylresten, so dass beispielsweise Terphenyl-, Quaterphenyl- oder Triphenyleneinheiten entstehen, die substituiert oder unsubstituiert sein können. Die Biphenyleinheiten können auch verbrückt sein, wie beispielsweise im Fall von Fluoren und dessen Derivaten.

Als Biphenyl können insbesondere die Verbindungen der Formeln 2 oder 3 als Reste A oder B eingesetzt werden:

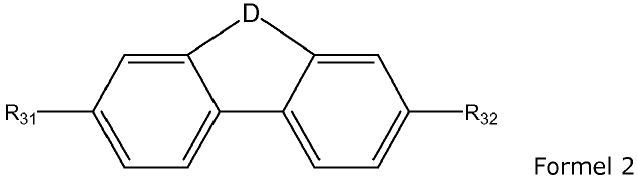

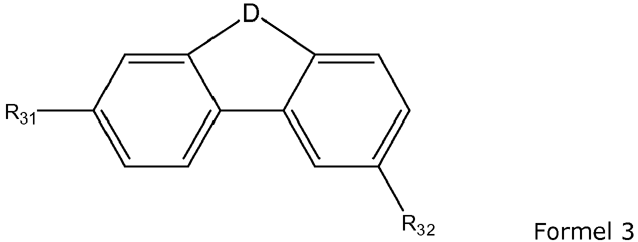

D kann Sauerstoff, Schwefel, Stickstoff oder Kohlenstoff sein und einfach (im Fall von Stickstoff) oder zweifach (im Fall von Kohlenstoff) mit Methyl, Ethyl, Biphenyl, 1-Naphthyl, 2-Naphthyl oder Phenyl substituiert sein. Es können also insbesondere die folgenden Reste vorliegen: 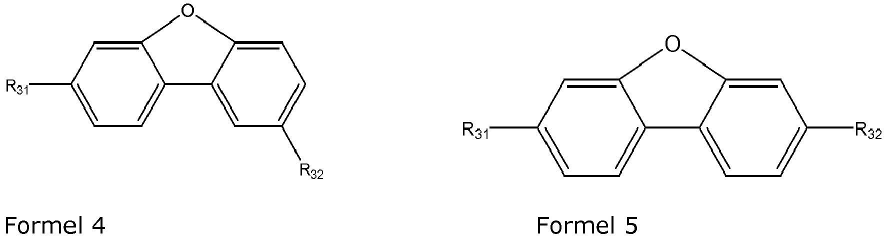 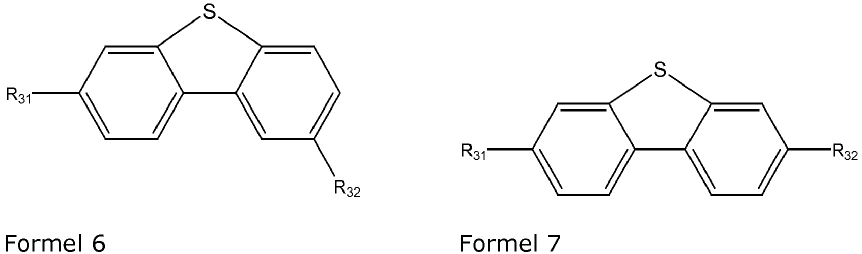 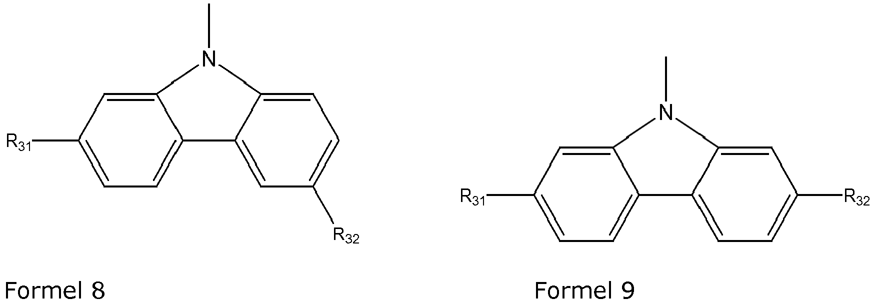 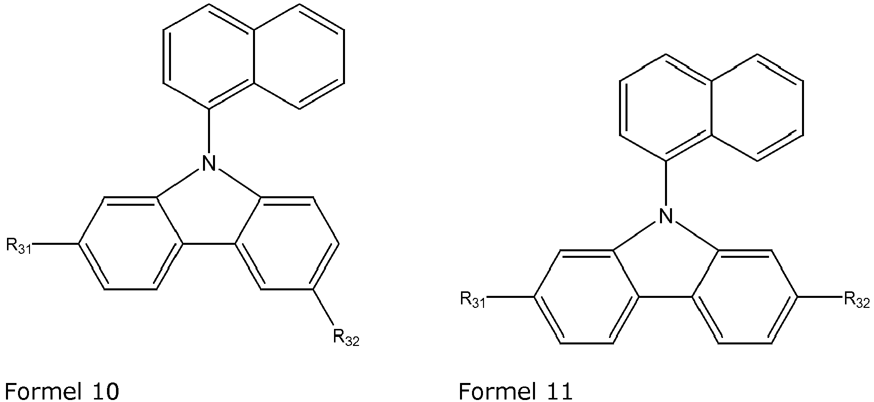 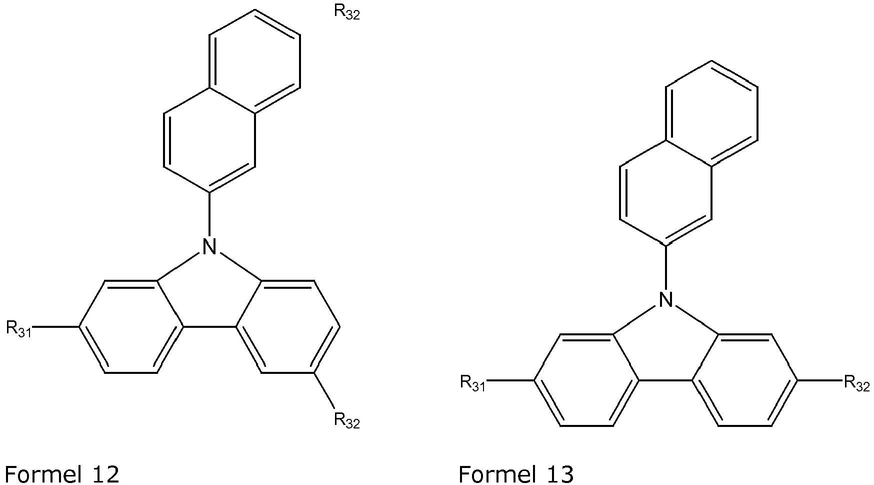 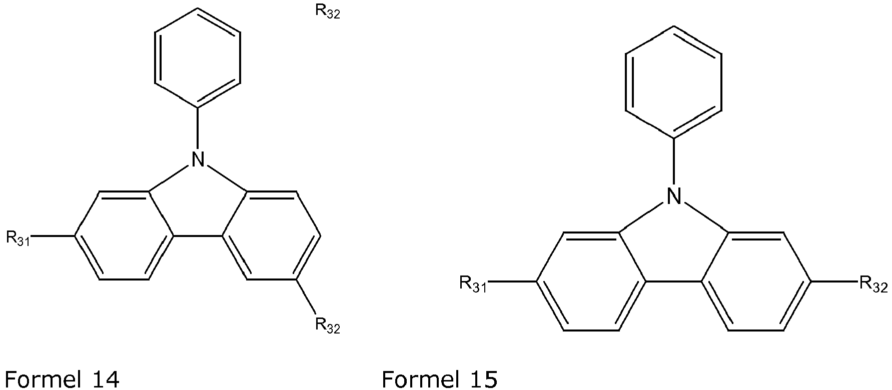 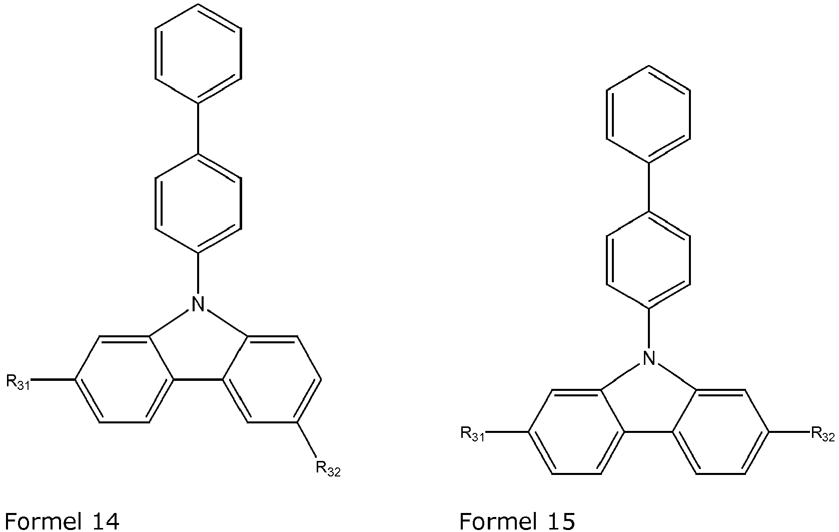 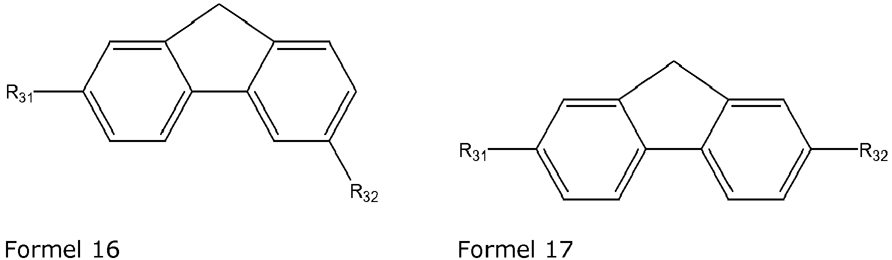 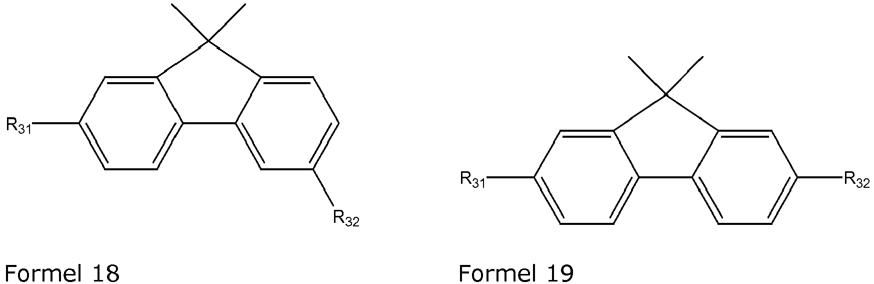 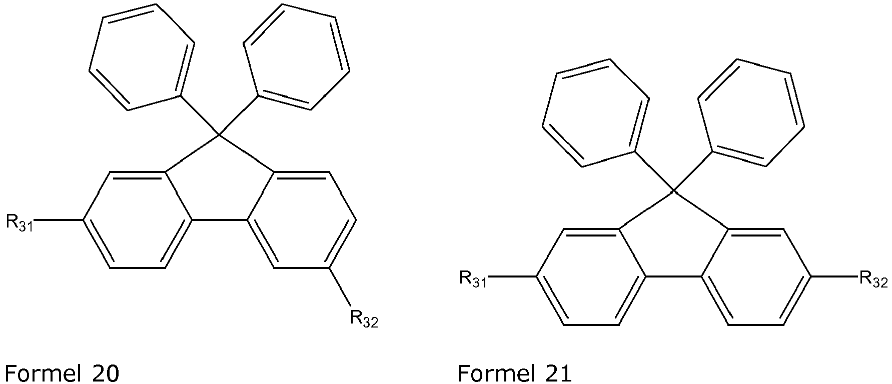 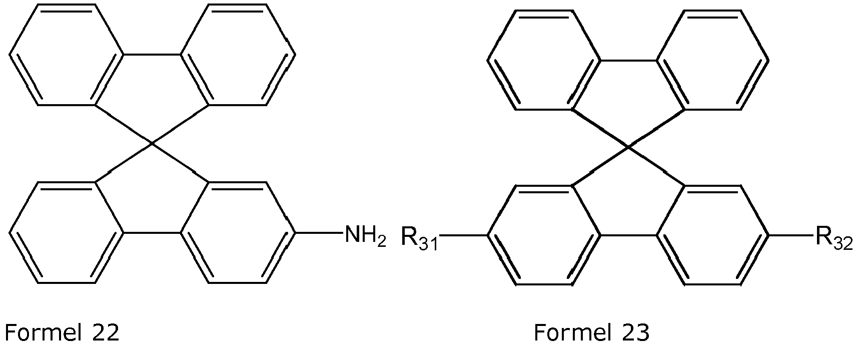 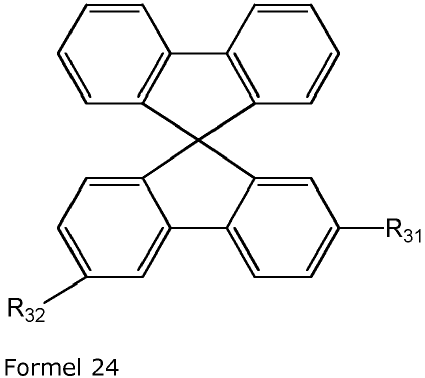
R31 kann Wasserstoff, Phenyl, Biphenyl oder Pyridyl sein.
R32 kann entweder wie oben beschrieben Halogen, eine primäre Amingruppe NH₂ oder ein Trifluormethylsulfonsäurerest sein, je nachdem ob der Rest der Formel 2 oder 3 als A oder B verwendet wird.
R32 kann aber auch ein Spacer sein, welcher zwischen A bzw. B und X bzw. NH₂ angeodnet ist. Geeignete Spacer sind beispielsweise 1,4-Phenyl, 1,4-(6-Methyl)phenyl, 1,4-(5-Methyl)phenyl, 4,4'-Biphenyl, 2,6-Naphthyl oder 1,4-Naphthyl. Dies sind insbesondere 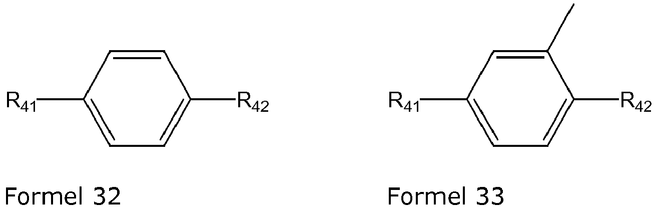 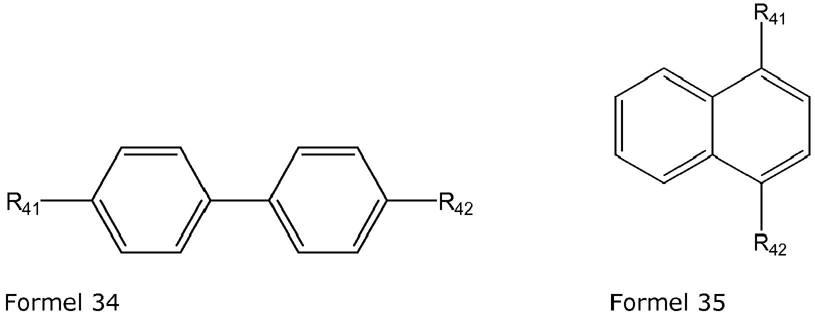 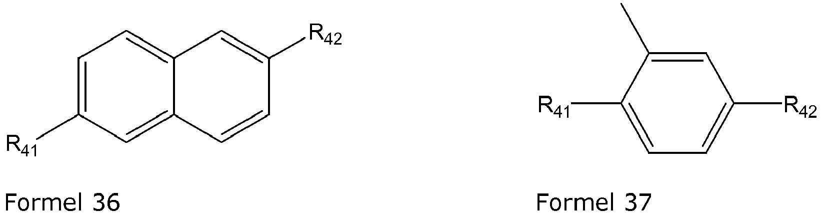
R41 ist hierbei eine Struktur der Formeln 2 bis 22, R42 kann Halogen wie Fluor, Chlor, Brom, Iod oder Astat, eine primäre Amingruppe NH₂ oder ein Trifluormethylsulfonsäurerest sein.

In einer spezifischen Ausgestaltung der Erfindung werden die folgenden Verbindungen verwendet, die Verbindung der Formel 131 als Amin der Formel A-NH₂ und die Verbindung der Formel 132 als Halogenaromat der Formel B-X

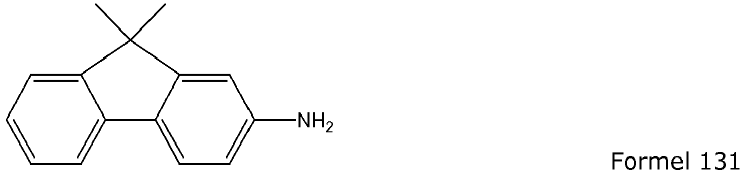

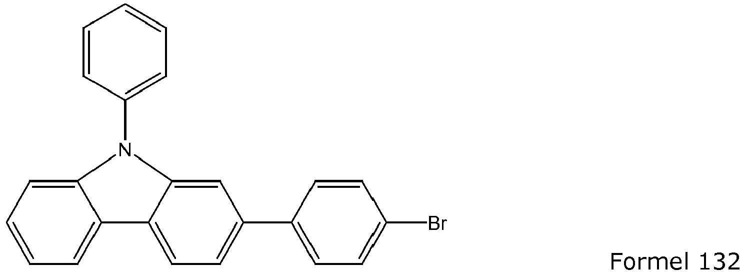

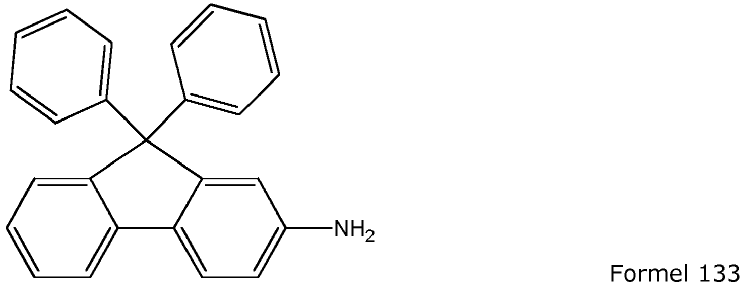

Die Ausgangsverbindungen, das primäre aromatische Amin A-NH₂ und der Halogenaromat X-B, werden äquimolar eingesetzt. Gegebenenfalls kann der Halogenaromat oder das Amin auch in einem Überschuss bis zum 1,1-fachen oder 1,2-fachen des äquimolaren Verhältnisses eingesetzt werden.

Zur Herstellung des tertiären Amins N-ABC, welches genauer durch die folgende Strukturformel wiedergegeben wird, ist eine weitere Reaktion des sekundären Amins mit einem weiteren Halogenaromaten X-C erforderlich 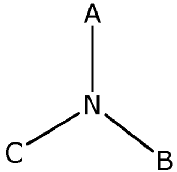 X-C ist wie X-B oben definiert und kann mit X-B identisch oder davon verschieden sein. Insbesondere ist C 4-Biphenyl, 3-Biphenyl oder 2-Fluorbiphen-4-yl, 2-Naphthyl, Carbazol-9-yl, Chinolin-6-yl, 2-Phenylchinazolin-4-yl.

C kann insbesondere auch die folgenden Strukturen aufweisen:

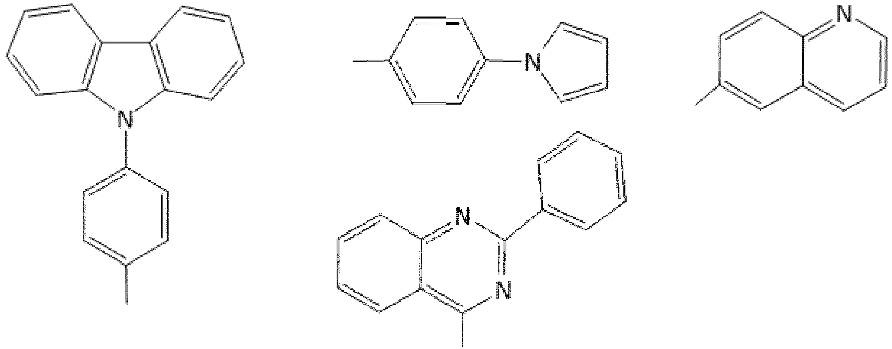

Die Palladiumkomplexe können auf im Prinzip bekannte Weise erhalten werden. Hierzu wird zunächst eine Palladiumverbindung in einem Lösungsmittel vorgelegt und anschließend der gewünschte Bis-(Dialkylphosphinoferrocen)-Ligand zugegeben, wobei mit 1,1'-Bis(diisopropylphosphino)ferrocen, 1,1'-Bis(diisobutylphosphino)ferrocen, 1,1'-Bis(di-tert.-butylphosphino)ferrocen gute Ergebnisse erzielbar sind. Anschließend kann zwischen 30 und 1000 Minuten, insbesondere 40 bis 400 Minuten oder 50 bis 120 Minuten gerührt werden. Die Reaktionstemperatur kann von etwa 10°C bis 100°C, insbesondere 15°C bis 50°C oder 20°C bis 30°C betragen.

Gute Ergebnisse lassen sich bereits durch Rühren bei Raumtemperatur für etwa eine Stunde erzielen.

Die Obergrenze der Temperatur hängt im Wesentlichen von der Siedetemperatur des Lösungsmittels ab, so dass hochsiedende Lösungsmittel für höhere Reaktionstemperaturen erforderlich sind und die oben angegebenen Temperaturobergrenzen nicht als starr, sondern in Abhängigkeit zur Siedetemperatur des Lösungsmittels zu sehen sein können.

Geeignete Lösungsmittel sind im Allgemeinen aprotische Lösungsmittel, wie Ether oder aromatische Lösungsmittel. Geeignet sind daher beispielsweise Diethylether, tert.-Butylethylether, tert.-Butylmethylether, Tetrahydrofuran oder Dioxan, aber auch Benzol, Toluol oder Xylol, aber auch Acetonitril kann eingesetzt werden.

Gute Ergebnisse sind durch Verwendung möglichst wasser- und sauerstofffreier Lösungsmittel erreichbar, die durch übliche Trocknungsverfahren der Lösemittel erhalten werden können.
Als Edukt für den Palladiumkomplex geeignete Palladiumverbindungen können Pd (0) und Pd(II)-Komplexe sein, wie z.B. Allylchloro[1,3-bis(2,4,6-trimethylphenyl)imidazol-2-ylidene]palladium(II), (Ethylenediamine) palladium(II)chlorid, Palladium(II)acetat, Palladium(II)chlorid, Palladiumpivalat, Palladium(II)acetylacetonat, Bis(benzonitrile)palladium(II) chloride, Bis(acetonitrile)dichlorpalladium(II), Diammindichlorpalladium(II), Dichlor(1,5-cyclooctadiene)palladium(II), Palladium(II)nitrat, Palladium(II)oxid, Palladium(II)oxidhydrat, H₂[PdCl₄], Diammindinitritopalladium(II), Palladium(II)sulfate, Tetraamminpalladium(II)sulfat [Pd(NH₃)₄]SO₄,Tetraamminpalladium(II) hydrogencarbonat, Tetraamminpalladium(II)chlorid [Pd(NH₃)₄]Cl₂, Kaliumtetrachlorpalladat(II) K₂[PdCl₄], Natriumtetrachlorpalladat(II) Na₂[PdCl₄], Ammoniumtetrachlorpalladate(II) (NH₄)₂[PdCl₄], Tetraamminpalladium(II)nitrat, 1,3-Divinyl-1,1,3,3-tetramethyldisiloxanpalladium(O) (auch als Pd-VTS, Pd-VS oder Palladium-VTS), Bis(dibenzylidenaceton)palladium(0) (Pd(dba)₂), (Pd₂(dba)₃), (Pd₂(dba)₃)·LM, wobei LM ein Lösungsmittel, insbesondere CHCl₃ oder CH₂Cl₂ ist. Auf diese Weise kann eine Katalysatorlösung erhalten werden.

Die Palladiumkomplexe des Typs PdX₁X₂L₁L₂ sind kommerziell erhältlich, beispielsweise bei Total Synthesis Ltd, deren Herstellung kann erfolgen wie beispielsweise beschrieben in Angewandte Chemie, International Edition 2012, 51(45), 11354-11357 sowie in Angewandte Chemie, International Edition 2014, 53(12), 3223-3226 sowie den Ergänzenden Informationen hierzu. Die Darstellung kann beispielsweise erfolgen, indem das Imidazolderivat, welches als L₁ eingesetzt wird, bei Raumtemperatur unter Inertgas und Wasser- und Sauerstoffausschluss in einem Ether, beispielsweise Tetrahydrofuran (THF), mit einer Base wie einem Alkalimetallalkoholat, zum Beispiel Kalium-tert.-Butanolat, bei Raumtemperatur umgesetzt wird. Die Reaktionszeit beträgt im Allgemeinen 1 bis 4 Stunden, oft sind etwa 2 Stunden ausreichend. Die Base wird vorteilhaft leicht überstöchiometrisch, also in Mengen von etwa 1,05 bis 1,2 Äquivalenten, insbesondere etwa 1,1 Äquivalenten eingesetzt. Anschließend wird in etwa die halbe molare Menge [(η³-allyl)Pd(µ-Cl)]₂ zugegeben, da es sich bei dieser Verbindung um ein Dimer handelt. Diese Reaktion wird ebenfalls bei Raumtemperatur unter Inertgas sowie Wasser- und Sauerstoffausschluss durchgeführt. Die Reaktionszeit beträgt etwa 20 bis 24 Stunden. Nach Filtrieren und Waschen des Niederschlags wird dieser für 1 bis 4 Tage, meist 2 Tage, bei Raumtemperatur in etherischer Salzsäure, also einer Lösung von Chlorwasserstoffgas in Diethylether, umgesetzt, wobei sich 2-molare etherische Salzsäure bewährt hat. Auf diese Weise wird der zweite Halogenligand am Palladiumatom eingeführt. Nach Entfernen des Lösemittels, beispielsweise durch Destillation, wird in einem weiteren Schritt unter Verwendung eines geeigneten Lösemittels, wie Halogenalkanen, beispielsweise Chloroform, Dichlormethan oder Tetrachlorethan, wiederaufgenommen und die erhaltene Lösung mit dem gewünschten Liganden L₂ versetzt. Geeignete Liganden sind oben beschrieben, vorteilhaft können Stickstoffheterocyclen wie insbesondere Pyridin, Pyrimidin oder Pyrazin sowie deren Derivate eingesetzt werden, insbesondere Pyridin oder 3-Chlorpyridin haben sich hierbei bewährt. Diese Reaktion wird für bis zu vier Stunden bei Raumtemperatur durchgeführt. Auch die letzten Schritte werden unter Inertgas, also Wasser- und Sauerstoffausschluss durchgeführt. Das Lösemittel kann nach der Reaktion entfernt und das Produkt säulenchromatographisch weiter gereinigt werden.

Nach der Herstellung des Palladiumkomplexes kann weiter verfahren werden, indem im Sinne einer Ein-Topf-Reaktion die weiteren Reaktanden, also das primäre aromatische Amin A-NH₂, der Halogenaromat X-B und die für die Hartwig-Buchwald-Kupplung erforderliche Base zu der Katalysatorlösung gegeben werden. Als Base sind Alkali- und Erdalkalimetallhydroxide geeignet, wie beispielsweise Lithiumhydroxid, Kaliumhydroxid oder Natriumhydroxid. Ebenso geeignet sind tertiäre organische Amine oder Alkoholate, wie Tributylamin, Triethylamin, Alkalimetallalkoholate wie Lithiumethanolat, Natriumethanolat oder Kaliumethanolat, Lithium-tert.-butanolat, Natrium-tert.-butanolat oder Kalium-tert.-butanolat, Lithiumbis(trimethylsilyl)amid, Natriumbis(trimethylsilyl)amid, Kaliumbis(trimethylsilyl)amid, einzeln oder in Kombination miteinander, als Base geeignet. Auch Alkalimetallsalze von BHT (2,6-Di-tert.-butylhydroxytoluol), insbesondere das Natrium- oder Kaliumsalz, haben sich bewährt, besonders beim Einsatz von Palladiumkomplexen des Typs PdX1X2L1L2 lassen sich hiermit gute Ergebnisse erzielen, insbesondere beim Einsatz dieses Komplexes als erster Katalysator.

Die Base kann in Bezug auf die gesamte molare Menge der Edukte, des primären aromatischen Amins A-NH₂ und des Halogenaromaten X-B bzw. des sekundären Amins AB-NH und des Halogenaromaten X-C, im Allgemeinen in Mengen von 40% bis 80%, insbesondere 50% bis 70% oder 55% bis 65% eingesetzt werden. Das bedeutet, wenn z.B. je 20 mmol des primären aromatischen Amins A-NH₂ und des Halogenaromaten X-B, zusammen also 40 mmol, verwendet werden, so kann die Base in Mengen von 16 mmol bis 32 mmol, insbesondere 20 mmol bis 28 mmol oder von 22 mmol bis 26 mmol eingesetzt werden. Gleiches gilt bei Verwendung des sekundären Amins AB-NH und des Halogenaromaten X-C.

Alternativ zu dieser Vorgehensweise können auch das primäre aromatische Amin A-NH₂, der Halogenaromat X-B und die Base mit einem Lösemittel versehen vorgelegt werden und die Katalysatorlösung in entsprechender Menge zudosiert werden.

Als Lösungsmittel für die Kupplungsreaktion zwischen dem aromatische Amin A-NH₂ und dem Halogenaromaten X-B bzw. dem sekundären Amins AB-NH und dem Halogenaromaten X-C geeignet sind die üblichen Lösungsmittel für Hartwig-Buchwald-Kupplungen, also Alkohole wie Ethanol, Propanol, Isopropanol, Butanol, Isobutanol, aber auch z.B. Ethylenglykol, Ether wie Diethylether, tert.-Butylethylether, tert.-Butylmethylether, Tetrahydrofuran, Dioxan Ethylenglykoldimethylether, Bis(2-methoxyethyl)ether, aromatische Lösungsmittel wie Benzol, Toluol oder Xylole, wie o-Xylol, p-Xylol, m-Xylol sowie deren Gemische, oder Kombinationen dieser Lösemittel. Bei den Lösemitteln ist darauf zu achten, dass diese unter den Bedingungen der Hartwig-Buchwald-Kupplung inert sind, da Amino- oder Halogengruppen die gewünschte Reaktion stören können.

Die Katalysatormenge, die für die Umsetzung nötig ist, liegt zwischen 0,01% bis 1,5 mol%, oder von 0,1 bis 1 mol%, oder von 0,2 mol% bis 0,8 mol%. Die Angabe in mol% bezieht sich auf die Gesamtmenge in mol der Edukte der jeweiligen Stufe, des primären aromatischen Amins A-NH₂ und des Halogenaromaten X-B bzw. des sekundären aromatischen Amins AB-NH und des Halogenaromaten X-C. Das sekundäre aromatische Amin AB-NH, welches als Zwischenprodukt zwangsläufig auftritt, kann vor der Umsetzung zum tertiären Amin ABC-N isoliert und gereinigt werden. Die Reaktion kann aber auch als Ein-Topf Reaktion durchgeführt werden, indem Base, das primäre aromatische Amin A-NH₂ und der Halogenaromat X-B in einem Lösemittel vorgelegt und in Gegenwart eines Katalysators umgesetzt werden. Anschließend kann der Halogenaromat X-C zugegeben und die Reaktion bei der zweiten Reaktionstemperatur fortgesetzt werden. Hierbei kann der zweite Katalysator ebenfalls nach der Reaktion zum sekundären Amin zugegeben werden. Vorzugsweise sind der erste und der zweite Katalysator in diesem Fall gleich. Insbesondere vorteilhaft ist in diesem Fall schon am Anfang, für die Reaktion des primären aromatischen Amins A-NH₂ und des Halogenaromaten X-B, eine ausreichende Katalysatormenge vorhanden, so dass die Umsetzung zum tertiären Amin ohne weitere Katalysatorzugabe, sondern lediglich durch Hinzufügen des Halogenaromaten X-C und Temperaturänderung auf die zweite Reaktionstemperatur bewirkt werden kann.

Die Umsätze der Edukte liegen mindestens bei 90%, insbesondere bei mindestens 95% oder bei mindestens 97%.

Die erste Reaktionstemperatur liegt im Allgemeinen bei 5°C bis 95°C, insbesondere bei 30°C bis 90°C, 50°C bis 80°C oder bei 55°C bis 70°C.

Die zweite Reaktionstemperatur ist höher als die jeweils verwendete erste Reaktionstemperatur und liegt im Allgemeinen bei etwa 90°C bis etwa 144°C, insbesondere bei 111°C bis 130°C oder bei 100°C bis 139°C oder 115°C bis 125°C oder 100°C bis 111°C.

Die Reaktionszeiten können sowohl für den Reaktionsschritt zum sekundären Amin als auch bei der Weiterreaktion zum tertiären Amin bei jeweils einer Stunde bis 36 Stunden, oder 4 Stunden bis 24 Stunden oder 6 Stunden bis 16 Stunden oder 8 Stunden bis 12 Stunden liegen.

In einer vorteilhaften Ausgestaltung wird neben dem Palladiumkomplex, wobei das Palladiumatom mit einem Bis-(Dialkylphosphinoferrocen)-Liganden der Formel 1 komplexiert ist, als Katalysator zusätzlich noch der entsprechende Ligand, also der im Palladiumkomplex verwendete Bis-(Dialkylphosphinoferrocen)-Ligand, zugegeben. Hier hat sich eine Zugabe im Verhältnis von Palladiumkomplex zu Bis-(Dialkylphosphinoferrocen)-Ligand im Bereich von 1 zu 10 bis 10 zu 1, oder von 1:5 bis 5:1, insbesondere von 2,5:1 bis 1:2,5, wie zum Beispiel 2 zu 1, als sinnvoll herausgestellt. Das Verhältnis bezieht sich auf die molaren Mengen von Palladiumkomplex und Bis-(Dialkylphosphinoferrocen)-Ligand. Dies bedeutet zum Beispiel, dass bei Verwendung von 0,2 mol% Palladiumkomplex 0,1 mol% Bis-(Dialkylphosphinoferrocen)-Ligand zugegeben werden.

Bei der oben beschriebenen Vorgehensweise kann die zusätzliche Menge Bis-(Dialkylphosphinoferrocen)-Ligand beispielsweise zu der Katalysatorlösung zugegeben werden, sowohl vor, während oder nach deren Herstellung aus den Ausgansprodukten, und diese dann bis zur Durchführung der Reaktion gelagert werden.

Alternativ können auch der Bis-(Dialkylphosphinoferrocen)-Ligand, das primäre aromatische Amin A-NH₂, der Halogenaromat X-B und die Base mit einem Lösemittel versehen gemeinsam vorgelegt werden und die Katalysatorlösung in entsprechender Menge zudosiert werden.

Alternativ kann aus der Katalysatorlösung aber auch ein fester Katalysator hergestellt werden. Hierzu wird der Katalysatorlösung ein Additiv zugegeben, welches einen schwer löslichen und gut kristallisierenden Palladiumkomplex bildet. Hierbei wird eine stöchiometrische Verbindung präzipitiert. Diese ist unter den Bedingungen der Präzipitation, also bei ausreichend niedrigem Lösemittelvolumen, ausreichend schwer löslich um auszufallen, jedoch gut genug löslich um wieder zur Herstellung einer Katalysatorlösung verwendet zu werden.

Bei der Herstellung des Palladiumkomplexes wird das Palladiumatom neben dem Bis-(Dialkylphosphinoferrocen)-Liganden auch von Liganden aus der zur Herstellung des Palladiumkomplexes eingesetzten Palladiumverbindung komplexiert, also beispielsweise von 1,3-Divinyl-1,1,3,3-tetramethyldisiloxan (auch als VTS oder VS bekannt), Bis(dibenzylidenaceton) (dba) oder Anderen.

Diese Palladiumkomplexe sind jedoch gut löslich. Aufgabe des Additivs ist es, das Palladiumatom zu komplexieren und hierdurch einen schwerer löslichen Palladiumkomplex zu bilden, der leicht abgetrennt und weiter verarbeitet werden kann. Geeignet als Additiv sind beispielsweise Naphthochinon, Maleinsäureanhydrid, Maleimid, Maleinsäurediethylester oder Norbornen, insbesondere Naphthochinon oder Maleimid. Es bilden sich dann schwer lösliche, gut kristallisierende Palladiumkomplexe, welche abfiltriert, gewaschen und getrocknet werden können. Hierbei wird eine stöchiometrische Verbindung präzipitiert. Diese ist unter den Bedingungen der Präzipitation, also bei ausreichend niedrigem Lösemittelvolumen, ausreichend schwer löslich um auszufallen, jedoch gut genug löslich um wieder zur Herstellung einer Katalysatorlösung verwendet zu werden, die zur Bereitstellung des ersten und/oder zweiten Katalysators geeignet ist.

Daher betrifft die vorliegende Patentanmeldung auch die Stoffe 1,1'-Bis(diisopropylphosphino)ferrocenpalladium(0)-maleimid [Pd(dippf)(maleimid)], 1,1'-Bis(diisopropylphosphino)ferrocenpalladium(0)-1,3-Divinyl-1,1,3,3,-tetramethyldisiloxan [Pd(dippf)(VTS)], 1,1'-Bis(diisopropylphosphino)ferrocenpalladium(0)-bis(dibenzylidenaceton) [Pd(dippf)(dba)], 1,1'-Bis(diisopropylphosphino)ferrocenpalladium(0)-naphthochinon [Pd(dippf)(Naphthochinon)], 1,1'-Bis(diisopropylphosphino)ferrocenpalladium(0)-maleinsäureanhydrid [Pd(dippf)(Maleinsäureanhydrid)], 1,1'-Bis(diisopropylphosphino)ferrocenpalladium(0)-maleinsäurediethylester [Pd(dippf)(Maleinsäurediethylester)], 1,1'-Bis(diisopropylphosphino)ferrocenpalladium(0)-Norbornen [Pd(dippf)(Norbornen)].

Das Palladiumatom (Palladium in der Oxidationsstufe 0) ist oft dreifach koordiniert und die Komplexe in der Regel trigonal-planar, so dass Verbindungen wie 1,3-Divinyl-1,1,3,3-tetramethyldisiloxan (VTS oder VS) oder Bis(dibenzylidenaceton), welche mehr als eine Doppelbindung enthalten, das Palladiumatom nur mit einer ihrer Doppelbindungen komplexieren. Die zweite Doppelbindung komplexiert entweder nicht oder ein weiteres Palladiumatom.

Die Struktur einiger dieser Verbindungen kann wie folgt dargestellt werden: 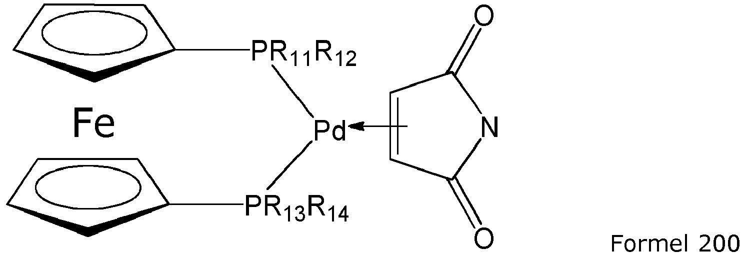 Die Reste R11 bis R14 können in Formel 200 gleich oder verschieden sein wie oben beschrieben und sind insbesondere Alkylreste mit einem bis fünf Kohlenstoffatomen. Sind R11 bis R14 Isopropylreste, so zeigt Formel 200 1,1'-Bis(diisopropylphosphino)ferrocenpalladium(0)-maleimid [Pd(dippf)(maleimid)].

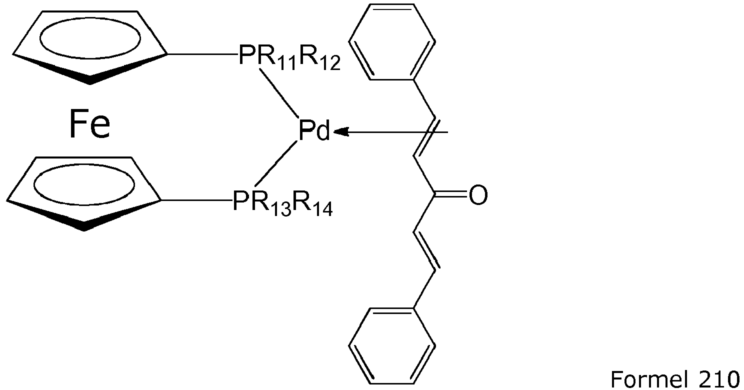

Die Reste R11 bis R14 können in Formel 210 gleich oder verschieden sein wie oben beschrieben und sind insbesondere Alkylreste mit einem bis fünf Kohlenstoffatomen. Sind R11 bis R14 Isopropylreste, so zeigt Formel 210 1,1'-Bis(diisopropylphosphino)ferrocenpalladium(0)-bis(dibenzylidenaceton) [Pd(dippf)(dba)].

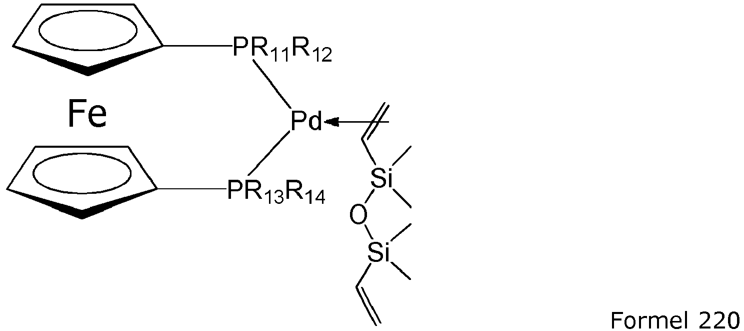

Die Reste R11 bis R14 können in Formel 220 gleich oder verschieden sein wie oben beschrieben und sind insbesondere Alkylreste mit einem bis fünf Kohlenstoffatomen. Sind R11 bis R14 Isopropylreste, so zeigt Formel 220 1,1'-Bis(diisopropylphosphino)ferrocenpalladium(0)-1,3-Divinyl-1,1,3,3,-tetramethyldisiloxan [Pd(dippf)(VTS)].

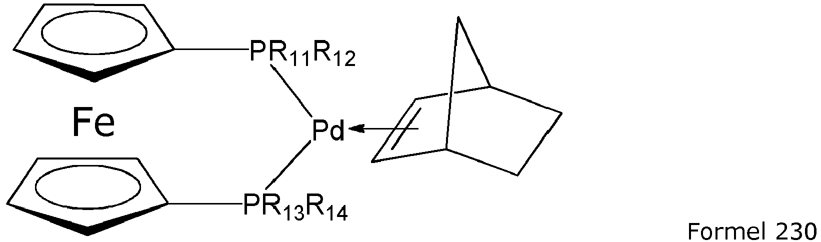

Die Reste R11 bis R14 können in Formel 230 gleich oder verschieden sein wie oben beschrieben und sind insbesondere Alkylreste mit einem bis fünf Kohlenstoffatomen. Sind R11 bis R14 Isopropylreste, so zeigt Formel 230 1,1'-Bis(diisopropylphosphino)ferrocenpalladium(0)-Norbornen [Pd(dippf)(Norbornen)].

Diese festen Palladiumkomplexe können ebenfalls wie oben beschrieben als Katalysator eingesetzt werden, wobei sie vorgelegt oder aber als Feststoff oder Lösung zu den Edukten und der Base zugegeben werden.

In einer vorteilhaften Ausgestaltung wird neben dem festen Palladiumkomplex zusätzlich noch der entsprechende Ligand, also der im festen Palladiumkomplex verwendete Bis-(Dialkylphosphinoferrocen)-Ligand, zugegeben.

Hier hat sich eine Zugabe im Verhältnis von festem Palladiumkomplex zu Bis-(Dialkylphosphinoferrocen)-Ligand im Bereich von 1 zu 10 bis 10 zu 1, oder von 1:5 bis 5:1, insbesondere von 2,5:1 bis 1: 2,5, wie zum Beispiel 2 zu 1, als sinnvoll herausgestellt. Das Verhältnis bezieht sich auf die molaren Mengen von festem Palladiumkomplex und Bis-(Dialkylphosphinoferrocen)-Ligand. Dies bedeutet zum Beispiel, dass bei Verwendung von 0,2 mol% festem Komplex 0,1 mol% Bis-(Dialkylphosphinoferrocen)-Ligand zugegeben werden. Der feste Palladiumkomplex kann aber auch mit zusätzlichem Bis-(Dialkylphosphinoferrocen)-Liganden im festen Zustand gemischt und so eine Pulvermischung erhalten werden. Diese Pulvermischung ist lagerstabil und kann einfach gehandhabt werden.

### Beispiele

### Beispiel 1: Herstellung der Katalysatorlösung

1,1'-Bis(diisopropylphosphino)ferrocen (dippf) (213 mg, 0.50 mmol) wurde in Diethylether (5 mL) gelöst und 1,3-Divinyl-1,1,3,3-tetrametyhldisiloxanpalladium(0) (Pd-VTS) in 2,4,6,8-Tetramethylcyclotetrasiloxan (0.50 mL, 0.50 mmol Pd) zugetropft. Das orange Gemisch wurde 1 Stunde bei Raumtemperatur gerührt.

### Beispiel 2: Herstellung des festen Katalysators [Pd(dippf)(maleimid)]

Es wurde zunächst wie in Beispiel 1 verfahren. Anschließend wurde Maleimid (99.1 mg, 1.00 mmol) in Diethylether (5 mL) zugegeben und für 10 Minuten in einem Ultraschallbad behandelt, woraufhin sich ein gelber Niederschlag bildete. Nach dem Absetzen des Feststoffes wurde die überstehende Lösung entfernt und der Rückstand mit Diethylether gewaschen (3 x 5 mL). Durch Trocknen im Vakuum (10⁻² mbar) konnte der gewünschte Palladium-Komplex als gelber Feststoff isoliert werden (291 mg, 0.47 mmol, 94%).

### Analytische Daten:

¹H NMR (400 MHz, Dioxan-*d*₈): *δ*= 7.84 (s, 1 H, N-H), 4.44 - 4.39 (m, 6 H, Ferrocen-H), 4.37 - 4.33 (m, 2 H, Ferrocen-H), 2.53 - 2.40 (m, 2 H), 2.35 - 2.20 (m, 2 H), 1.34 (d, J=7.0 Hz, 3 H), 1.30 (d, J=7.0 Hz, 3 H), 1.26 (d, *J*=7.3 Hz, 3 H), 1.22 (d, J=7.3 Hz, 6 H), 1.18 (d, J=7.3 Hz, 3 H), 1.13 (d, J=7.3 Hz, 3 H), 1.09 ppm (d, J=7.3 Hz, 3 H).

³¹P-NMR (162 MHz, Dioxan-*d*₈): *δ*=: 38.87 ppm (s, 2 P).

### Beispiel 3: Synthesen im präparativen Maßstab

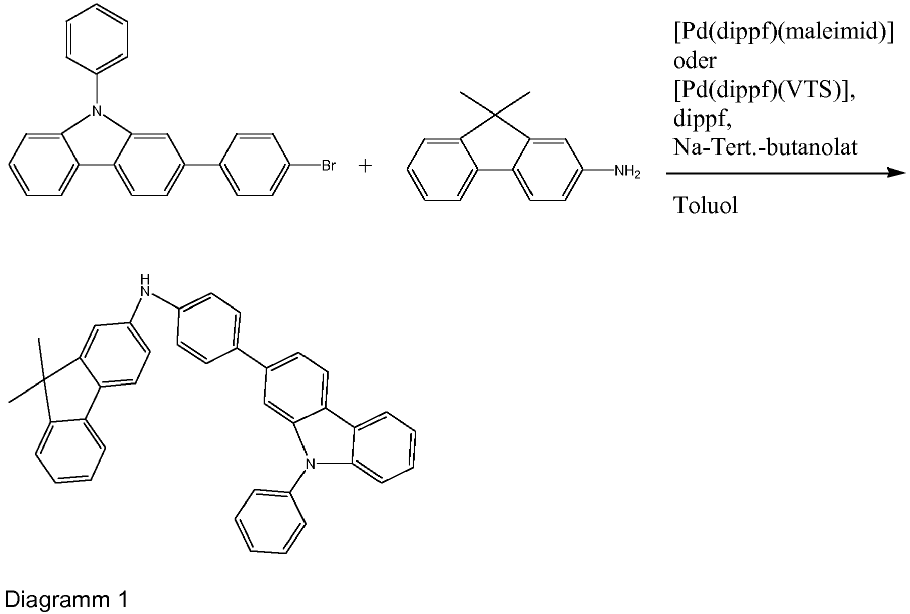

### Beispiel 3a: Verwendung von [Pd(dippf)(maleimid)] als Palladiumkomplex

In einem trockenen Dreihalskolben mit Rückflusskühler, Gaseinlass, Überdruckventil und Magnetrührstab wurden das Arylbromid 132 (7.97 g, 20.0 mmol), das Amin 131 (4.17 g, 20.0 mmol), 1,1'-Bis(diisopropylphosphino)ferrocen (8.5 mg, 0.02 mmol) und Natrium-*tert-*butanolat (2.35 g, 24.0 mmol) vorgelegt und durch mehrfaches Evakuieren und Begasen unter eine Stickstoffatmosphäre gebracht. Anschließend wurden Toluol (30 mL) und [Pd(dippf)(maleimid)] (24.9 mg, 0.04 mmol, aus Beispiel 2) in Toluol (10 mL) zugegeben und das Gemisch für 20 Stunden auf 70 °C erhitzt. Die Reaktion wurde durch Dünnschichtchromatographie verfolgt und festegestellt, dass nach dieser Zeit der volle Umsatz erreicht war. Nach dem Abkühlen auf Raumtemperatur wurde Wasser (60 mL) zugegeben und mit Dichlormethan (150 mL) extrahiert. Die organische Phase wurde abgetrennt, über Magnesiumsulfat (5 g) getrocknet und durch basisches Aluminiumoxid (10 g) filtriert. Das Lösungsmittel wurde im Vakuum entfernt (40 °C, 10 mbar) und der leicht gelbe Rückstand mit Diethylether (3 x 20 mL) gewaschen. Nach dem Trocknen im Vakuum (2h, 10⁻² mbar) wurde das Produkt als farbloser Feststoff erhalten (10.2 g, 19.4 mmol, 97%).

¹H NMR (400 MHz, Chloroform-*d*): δ= 8.41 (d, *J*=1.3 Hz, 1 H), 8.26 (d, *J*=7.8 Hz, 1 H), 7.75 - 7.60 (m, 9 H), 7.55 - 7.44 (m, 5 H), 7.41 - 7.21 (m, 6 H), 7.13 (d, *J*=7.0 Hz, 1 H), 5.91 (s, 1 H), 1.55 ppm (s, 6 H).

¹³C NMR (101 MHz, Chloroform-*d*): *δ*= 155.3, 153.1, 142.6, 142.0, 141.3, 140.0, 139.3, 137.7, 134.6, 133.2, 132.5, 129.9 (2 C), 128.1, 127.4 (2 C), 127.0 (2 C), 126.9, 126.0 (2 C), 125.0, 123.9, 123.5 (2 C), 122.4, 120.8, 120.3, 120.0, 119.9, 119.1, 118.1, 116.8, 112.2, 110.0, 109.9, 46.8, 27.2 ppm (2 C).

Elementaranalyse: Berechnet für C₃₉H₃₀N₂: C 88.94, H 5.74, N 5.32; gefunden: C 88.64, H 5.91, N 5.22.

### Beispiel 3b: Verwendung von [Pd(dippf)(VTS)] als Palladiumkomplex

Für diese Reaktion wurde eine Katalysatorstammlösung ähnlich wie in Beispiel 1 beschrieben aus 1,1'-Bis(diisopropylphosphino)ferrocen (213,8 mg, 0.50 mmol), Toluol (0.5 mL) und einem Gemisch aus 1,3-Divinyl-1,1,3,3-tetrametyhldisiloxanpalladium(0) (Pd-VTS) in 2,4,6,8-Tetramethylcyclotetrasiloxan (0,5 mL, 10.87% Palladium) hergestellt, verwendet. Das Gemisch wurde eine Stunde bei Raumtemperatur gerührt.

In einem trockenen Dreihalskolben mit Rückflusskühler, Gaseinlass, Überdruckventil und Magnetrührstab wurden das Arylbromid 132 (7.97 g, 20.0 mmol), das Amin 131 (4.17 g, 20.0 mmol) und Natrium-*tert*-butanolat (2.35 g, 24.0 mmol) vorgelegt und durch Evakuieren und Begasen unter eine Stickstoffatmosphäre gebracht. Anschließend wurden Toluol (40 mL) und die Katalysatorstammlösung (100 µL, 91.8 mg, 0.04 mmol Palladium) zugegeben und das Gemisch für 20 Stunden auf 70 °C erhitzt. Die Reaktion wurde durch Dünnschichtchromatographie verfolgt und festgestellt, dass nach dieser Zeit der volle Umsatz erreicht war. Nach dem Abkühlen auf Raumtemperatur wurde Wasser (60 mL) zugegeben und mit Dichlormethan (150 mL) extrahiert. Die organische Phase wurde abgetrennt, über Magnesiumsulfat (5 g) getrocknet und durch basisches Aluminiumoxid (10 g) filtriert. Das Lösungsmittel wurde im Vakuum entfernt (40 °C, 10 mbar) und der leicht gelbe Rückstand mit Ether (3 x 20 mL) gewaschen. Nach dem Trocknen im Vakuum (2h, 10⁻² mbar) wurde das Produkt als farbloser Feststoff erhalten (9,71 g, 18.4 mmol, 92%).

Die Reaktionsgleichung ist in Diagramm 1 abgebildet. Eine Mehrfacharylierung des primären Amins 131 durch das Arylbromid 132 wurde in keinem der Beispiele beobachtet.

### Beispiele 4a bis 4i:

### Allgemeine Vorschrift:

Alle Versuche wurden in 20 mL Headspace-Vials für die Gaschromatographie durchgeführt, die mit Aluminium-Bördelkappen mit Teflon-beschichteten Butylgummi-Septen verschlossen wurden (beides erhältlich z.B. bei der Firma *VWR*). Zur Temperierung der Gefäße wurden 8 cm hohe zylindrische Aluminiumblöcke verwendet, die in ihrem Durchmesser genau dem der Heizplatten von Labor-Magnetrührern (z.B. Heidolph Mr 2002) entsprechen. Diese Aluminiumblöcke wurden mit zehn 7 cm tiefen Bohrungen vom Durchmesser der Reaktionsgefäße und einer Bohrung zur Aufnahme eines Temperaturfühlers versehen.
Zum gleichzeitigen Evakuieren und Rückfüllen von jeweils zehn Gefäßen wurden Vakuumverteiler zum Anschluss an die Schlenk-Linie angefertigt. Dazu wurden jeweils zehn vakuumfeste 3 mm Teflonschläuche jeweils an einem Ende mit Adaptern zur Aufnahme von Luer-Lock-Spritzennadeln verbunden und dem anderen Ende an ein Stahlrohr angeschlossen, das über einen Vakuumschlauch mit der Schlenk-Linie verbunden werden kann.

Zur Durchführung der Versuche wurden das Arylbromid (1.00 mmol), das entsprechende primäre Amin (1.00 mmol) und Natrium-*tert*-butanolat (118 mg, 1.20 mmol) an der Luft in die Reaktionsgefäße eingewogen, 20 mm Magnet-Rührkerne zugegeben und die Gefäße mittels einer Bördelzange mit Septumkappen luftdicht verschlossen. Jeweils 10 Reaktionsgefäße wurden in die Bohrungen eines Aluminiumblocks gesteckt und über Hohlnadeln, die durch die Septenkappen gebohrt wurden, mit dem Vakuumverteiler verbunden.

Die Reaktionsgefäße wurden danach gemeinsam dreimal hintereinander evakuiert und mit Stickstoff begast. Nachdem die Reaktionsgefäße auf diese Weise mit einer Inertgasatmosphäre versehen waren, wurde an der Vakuumlinie über ein Überdruckventil ein Druckausgleich mit der Außenatmosphäre hergestellt. Mit Hilfe einer Spritze wurde eine Stammlösung von [Pd(dippf)(maleimid)] (1.24 mg, 0.002 mmol) und dippf (0.85 mg, 0.002 mmol) in Toluol (2 mL) durch die Septenkappen hindurch injiziert. Danach wurde der Aluminiumblock auf 70 °C gebracht und die Nadeln des Vakuumverteilers entfernt.

Nach 20 h Reaktionszeit wurden die Gefäße nach dem Abkühlen vorsichtig geöffnet und das Reaktionsmedium mit Dichlormethan (30 mL) und Wasser (30 mL) verdünnt. Die wässrige Phase wurde mit 1N Salzsäure auf pH = 7 gestellt, von der organischen Phase getrennt und mit Dichlormethan (2 x 20 mL) extrahiert. Die vereinigten organischen Phasen wurden über Magnesiumsulfat getrocknet, filtriert und schließlich Dünnschichtchromatographisch untersucht.

Das Lösungsmittel wurde im Vakuum entfernt (40°C, 500 mbar) und das verbliebene Rohprodukt säulenchromatographisch (basisches Al₂O₃, Diethylether: Hexan oder Ethylacetat: Hexan) gereinigt.

### Beispiel 4a: Synthese von Verbindung 3a:

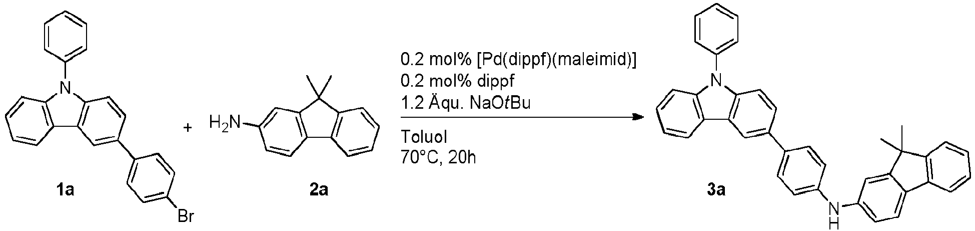

Nach der allgemeinen Vorschrift wurde Verbindung **3a** ausgehend von **1a** (398 mg, 1.00 mmol) und **2a** (209 mg, 1.00 mmol) dargestellt und konnte säulenchromatographisch (Al₂O₃, Diethylether:Hexan = 1:1) in 94% Ausbeute (496 mg, 0.94 mmol) isoliert werden. ¹H NMR (400 MHz, Chloroform-*d*): *δ*= 8.41 (d, *J*=1.3 Hz, 1 H), 8.26 (d, *J*=7.8 Hz, 1 H), 7.75 - 7.60 (m, 9 H), 7.55 - 7.44 (m, 5 H), 7.41 - 7.21 (m, 6 H), 7.13 (d, *J*=7.0 Hz, 1 H), 5.91 (s, 1 H), 1.55 ppm (s, 6 H). ¹³C NMR (101 MHz, Chloroform-*d*): *δ*= 155.3, 153.1, 142.6, 142.0, 141.3, 140.0, 139.3, 137.7, 134.6, 133.2, 132.5, 129.9 (2 C), 128.1, 127.4 (2 C), 127.0 (2 C), 126.9, 126.0 (2 C), 125.0, 123.9, 123.5 (2 C), 122.4, 120.8, 120.3, 120.0, 119.9, 119.1, 118.1, 116.8, 112.2, 110.0, 109.9, 46.8, 27.2 ppm (2 C). CHN: Berechnet für C₃₉H₃₀N₂: C 88.94, H 5.74, N 5.32; gefunden: C 88.79, H 5.86, N 5.19.

### Beispiel 4b: Synthese von Verbindung 3b:

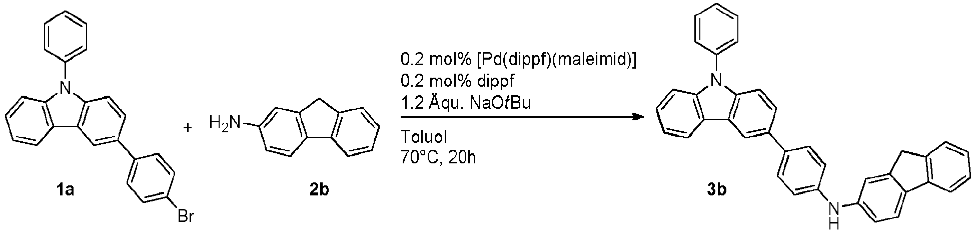

Nach der allgemeinen Vorschrift wurde Verbindung **3b** ausgehend von **1a** (398 mg, 1.00 mmol) und **2b** (181 mg, 1.00 mmol) dargestellt und konnte säulenchromatographisch (Al₂O₃, Diethylether:Hexan = 1:1) in 92% Ausbeute (460 mg, 0.92 mmol) isoliert werden. ¹H NMR (400 MHz, DMSO-*d₆*): δ = 8.54 (d, *J*=1.5 Hz, 1H), 8.44 (s, 1H), 8.35 (d, *J*=7.8 Hz, 1H), 7.77-7.63 (m, 9H), 7.55 (m, 1H), 7.51 (d, *J*=7.5 Hz, 1H), 7.47 -7.36 (m, 4H), 7.31 (m, 2H), 7.26 (d, *J*=8.8 Hz, 2H), 7.20 (dt, *J*=7.5 Hz, 1.3 Hz, 1H), 7.15 (dd, *J*=8.3 Hz, 2.0 Hz, 1H), 3.87 ppm (s, 2H). ¹³C NMR (101 MHz, DMSO-*d₆*): δ = 144.6, 142.7, 142.3, 142.2, 141.5, 140.5, 139.1, 136.9, 133.3, 132.6, 132.2, 130.2 (2C), 127.6, 127.5 (2C), 126.7, 126.6 (2C), 126.4, 125.3, 124.9, 124.7, 123.4, 123.0, 120.8, 120.7, 120.1, 118.8, 117.6, 117.4 (2C), 117.3, 115.9, 113.1, 110.0, 109.7 ppm. CHN: Berechnet für C₃₇H₂₆N₂: C 89.13, H 5.26, N 5.62; gefunden: C 88.83, H 5.25, N 5.54.

### Beispiel 4c: Synthese von Verbindung 3c:

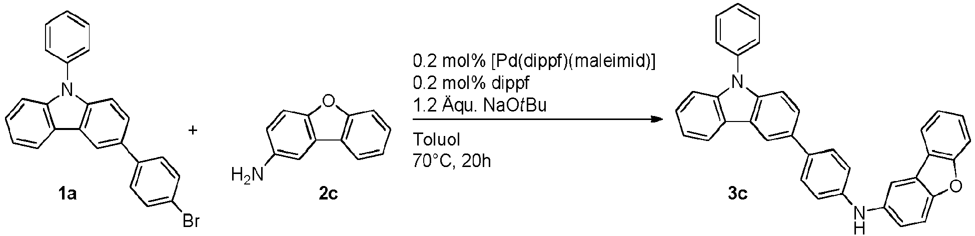

Nach der allgemeinen Vorschrift wurde Verbindung **3c** ausgehend von **1a** (398 mg, 1.00 mmol) und **2c** (183 mg, 1.00 mmol) dargestellt und konnte säulenchromatographisch (Al₂O₃, Diethylether: Hexan = 1:1) in 88% Ausbeute (442 mg, 0.88 mmol) isoliert werden. ¹H NMR (400 MHz, DMSO-*d₆*): δ = 8.72 (s, 1H), 8.55 (d, *J*=1.5 Hz, 1H), 8.35 (d, *J*=7.8 Hz, 1H), 7.99-7.93 (m, 2H), 7.77-7.62 (m, 7H), 7.60 (d, *J*=8.0 Hz, 1H), 7.54 (m, 1H), 7.47-7.27 (m, 9H), 7.16 ppm (dd, *J*=8.5 Hz, 1.8 Hz, 1H). ¹³C NMR (101 MHz, DMSO-*d₆*): δ = 157.1, 155.3, 144.0, 141.6, 140.5, 139.2, 136.9, 133.0, 132.5, 130.2 (2C), 127.6, 127.5 (2C), 126.6 (2C), 126.4, 125.6, 124.7, 124.2, 123.4, 123.0 (2C), 121.6, 120.8, 120.1, 119.7, 118.0 (2C), 117.7, 115.5, 113.1, 111.2, 110.0, 109.7, 97.9 ppm. CHN: Berechnet für C₃₆H₂₄N₂O: C 86.38, H 4.83, N 5.60; gefunden: C 86.02, H 5.04, N 5.43.

### Beispiel 4c: Synthese von Verbindung 3d:

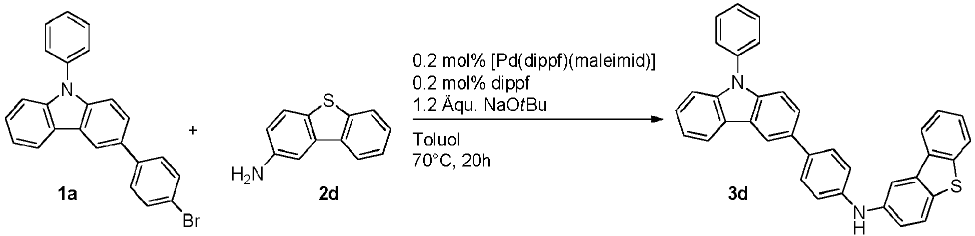

Nach der allgemeinen Vorschrift wurde Verbindung **3d** ausgehend von **1a** (398 mg, 1.00 mmol) und **2d** (199 mg, 1.00 mmol) dargestellt und konnte säulenchromatographisch (Al₂O₃, Diethylether:Hexan = 1:1) in 96% Ausbeute (496 mg, 0.96 mmol) isoliert werden. ¹H NMR (400 MHz, DMSO-*d₆*): δ = 8.54 (d, *J*=1.5 Hz, 1H), 8.51 (s, 1H), 8.35 (d, *J*=7.5 Hz, 1H), 8.25 (m, 1H), 8.07 (d, *J*=2.0 Hz, 1H), 7.99 (m, 1H), 7.89 (d, *J*=8.5 Hz, 1H), 7.75-7.63 (m, 7H), 7.55 (m, 1H), 7.48 (m, 2H), 7.45-7.37 (3H), 7.36-7.27 ppm (m, 4H). ¹³C NMR (101 MHz, DMSO-*d₆*): δ = 142.7, 141.0, 140.5, 139.5, 139.1, 136.9, 136.0, 134.9, 132.6, 132.1, 130.2 (2C), 129.7, 127.6, 127.5 (2C), 126.9, 126.6 (2C), 126.4, 124.7, 124.5, 123.6, 123.4, 123.1, 123.0, 121.9, 120.8, 120.1, 119.0, 117.6, 116.8 (2C), 110.0, 109.7, 109.2 ppm. CHN: Berechnet für C₃₆H₂₄N₂S: C 83.69, H 4.68, N 5.42, S 6.21; gefunden: C 83.40, H 4.76, N 5.35, S 6.31.

### Beispiel 4d: Synthese von Verbindung 3e:

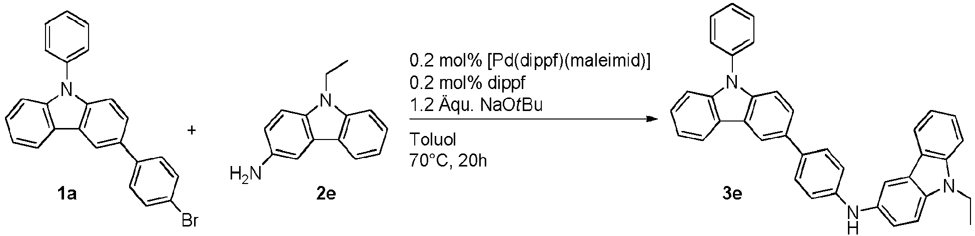

Nach der allgemeinen Vorschrift wurde Verbindung **3e** ausgehend von **1a** (398 mg, 1.00 mmol) und **2e** (221 mg, 1.00 mmol) dargestellt und konnte säulenchromatographisch (Al₂O₃, Ethylacetat: Hexan = 1:2) in 95% Ausbeute (502 mg, 0.95 mmol) isoliert werden. ¹H NMR (400 MHz, DMSO-*d₆*): δ = 8.50 (d, *J*=1.5 Hz, 1H), 8.33 (d, *J*=7.8 Hz, 1H), 8.14 (s, 1H), 8.11 (d, *J*=7.5 Hz, 1H), 7.96 (d, *J*=2.0 Hz, 1H), 7.72 -7.61 (m, 7H), 7.58-7.51 (m, 3H), 7.46-7.37 (m, 4H), 7.35 -7.27 (m, 2H), 7.17-7.11 (m, 3H), 4.41 (q, *J*=7.0 Hz, 2H), 1.31 ppm (t, *J*=7.0 Hz, 3H). ¹³C NMR (101 MHz, DMSO-*d₆*): δ = 144.6, 142.7, 142.3, 142.2, 141.5, 140.5, 139.1, 136.9, 133.3, 132.6, 132.2, 130.2 (2C), 127.6, 127.5 (2C), 126.7, 126.6 (2C), 126.4, 125.3, 124.9, 124.7, 123.4, 123.0, 120.8, 120.7, 120.1, 118.8, 117.6, 117.4 (2C), 117.3, 115.9, 113.1, 110.0, 109.7, 36.5 ppm. CHN: Berechnet für C₃₈H₂₉N₃: C 86.50, H 5.54, N 7.96; gefunden: C 86.32, H 5.63, N 7.90.

### Beispiel 4e: Synthese von Verbindung 3f:

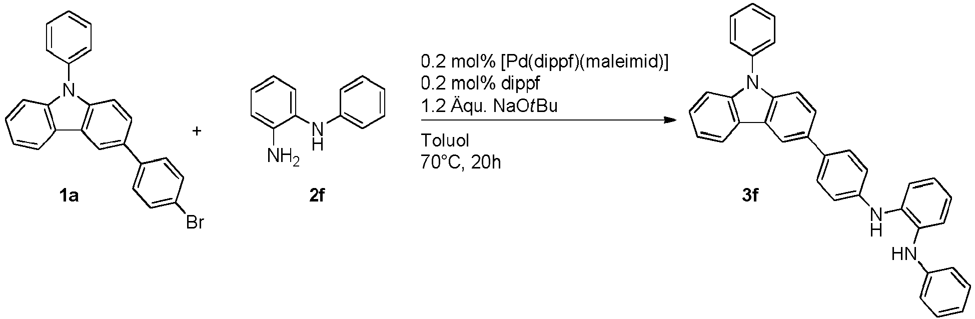

Nach der allgemeinen Vorschrift wurde Verbindung **3f** ausgehend von **1a** (398 mg, 1.00 mmol) und **2f** (188 mg, 1.00 mmol) dargestellt und konnte säulenchromatographisch (Al₂O₃, Ethylacetat: Hexan = 1:2) in 84% Ausbeute (423 mg, 0.84 mmol) isoliert werden. ¹H NMR (400 MHz, DMSO-*d₆*): *δ* = 8.49 (d, *J*=1.5 Hz, 1H), 8.33 (d, *J*=7.8 Hz, 1H), 7.72-7.61 (m, 7H), 7.53 (m, 1H), 7.46-7.37 (m, 4H), 7.36 (s, 1H), 7.34-7.26 (m, 3H), 7.19 (m, 2H), 7.10 (d, *J*=8.5 Hz, 2H), 6.99 (dd, *J*=8.7 Hz, 0.9 Hz, 2H), 6.94 (m, 2H), 6.76 ppm (m, 1H). ¹³C NMR (101 MHz, DMSO-*d₆*): *δ* = 144.5, 143.4, 140.5, 139.0, 136.9, 134.6 (2C), 132.7, 131.6, 130.2 (2C), 129.0 (2C), 127.6, 127.3 (2C), 126.6 (2C), 126.3, 124.6, 123.4, 123.0, 122.1, 122.0, 120.8, 120.1, 119.9 (2C), 119.0, 117.5, 116.7 (2C), 116.2 (2C), 109.9, 109.6 ppm. CHN: Berechnet für C₃₆H₂₇N₃: C 86.20, H 5.43, N 8.38; gefunden: C 85.82, H 5.62, N 8.22.

### Beispiel 4f: Synthese von Verbindung 3g:

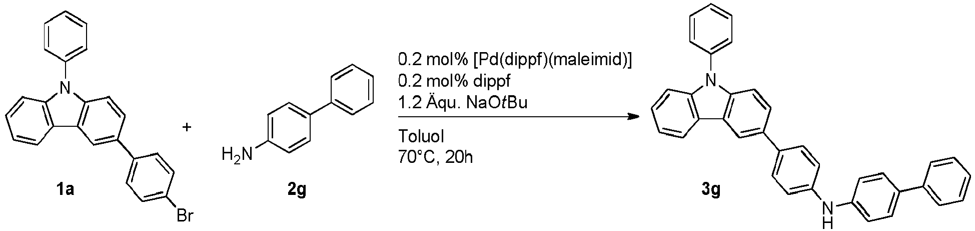

Nach der allgemeinen Vorschrift wurde Verbindung **3g** ausgehend von **1a** (398 mg, 1.00 mmol) und **2g** (169 mg, 1.00 mmol) dargestellt und konnte säulenchromatographisch (Al₂O₃, Diethylether:Hexan = 1:1) in 95% Ausbeute (462 mg, 0.95 mmol) isoliert werden. ¹H NMR (400 MHz, DMSO-*d₆*): δ = 8.53 (d, *J*=1.3 Hz, 1H), 8.47 (s, 1H), 8.35 (d, *J*=7.5 Hz, 1H), 7.74-7.53 (m, 12H), 7.47-7.38 (m, 5H), 7.34-7.19 ppm (m, 6H). ¹³C NMR (101 MHz, DMSO-*d₆*): δ = 143.0, 142.9, 141.9, 140.5, 140.1, 139.1, 136.9, 132.6, 132.5, 131.2, 130.2 (2C), 128.9 (2C), 127.6, 127.5 (2C), 127.4 (2C), 126.6 (2C), 126.4 (2C), 125.8 (2C), 124.7, 123.4, 123.0, 120.8, 120.1, 117.7, 117.5 (2C), 116.8, 110.0, 109.7 ppm. CHN: Berechnet für C₃₆H₂₆N₂: C 88.86, H 5.39, N 5.76; gefunden: C 88.49, H 5.39, N 5.68.

### Beispiel 4g: Synthese von Verbindung 3i:

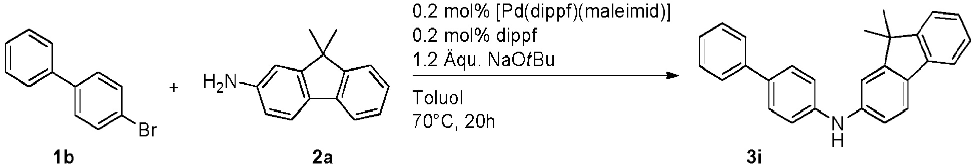

Nach der allgemeinen Vorschrift wurde Verbindung **3i** ausgehend von **1b** (259 mg, 1.00 mmol) und **2a** (209 mg, 1.00 mmol) dargestellt und konnte säulenchromatographisch (Al₂O₃, Diethylether: Hexan = 1:2) in 89% Ausbeute (323 mg, 0.89 mmol) isoliert werden. ¹H NMR (400 MHz, Chloroform-*d*): *δ* = 7.72-7.63 (m, 4H), 7.59 (m, 2H), 7.51-7.44 (m, 3H), 7.37 (m, 2H), 7.32 (t, *J*=7.0 Hz, 1H), 7.25 (br. s, 1H), 7.22 (d, *J*=8.3 Hz, 2H), 7.13 (br. d, *J*=7.5 Hz, 1H), 5.92 (br. s, 1H), 1.54 ppm (s, 6H). ¹³C NMR (101 MHz, Chloroform-*d*): *δ* = 155.3, 153.1, 142.7, 142.1, 140.8, 139.2, 133.5, 132.8, 128.7 (2C), 128.0 (2C), 126.9, 126.6, 126.5 (2C), 126.1, 122.4, 120.8, 119.1, 117.6 (2C), 117.2, 112.6, 46.8, 27.2 ppm (2C). CHN: Berechnet für C₂₇H₂₃N: C 89.71, H 6.41, N 3.87; gefunden: C 89.69, H 6.27, N 3.84.

### Beispiel 4h: Synthese von Verbindung 3j:

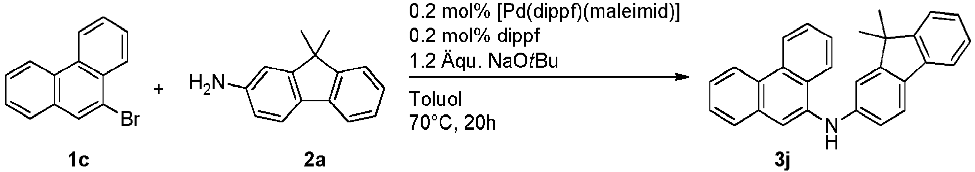

Nach der allgemeinen Vorschrift wurde Verbindung **3j** ausgehend von **1c** (268 mg, 1.00 mmol) und **2a** (209 mg, 1.00 mmol) dargestellt und konnte säulenchromatographisch (Al₂O₃, Diethylether: Hexan = 1:2) in 87% Ausbeute (335 mg, 0.87 mmol) isoliert werden. ¹H NMR (400 MHz, DMSO-*d₆*): δ = 8.86 (m, 1H), 8.72 (m, 1H), 8.42 (s, 1H), 8.37 (dd, *J*=8.2 Hz, 1.1 Hz, 1H), 7.77-7.65 (m, 5H), 7.63 (s, 1H), 7.51 (m, 2H), 7.45 (d, *J*=7.5 Hz, 1H), 7.32 (d, *J*=2.0 Hz, 1H), 7.27 (dt, *J*=7.5 Hz, 1.1 Hz, 1H), 7.20 (dt, *J*=7.3 Hz, 1.0 Hz, 1H), 7.13 (dd, *J*=8.3 Hz, 2.0 Hz, 1H), 1.40 ppm (s, 6H). ¹³C NMR (101 MHz, DMSO-*d₆*): δ = 154.7, 152.7, 144.6, 139.0, 137.8, 132.5, 131.0, 130.8, 127.5, 127.1, 127.1, 127.0, 126.9, 126.6, 126.4, 125.8, 124.5, 123.3, 123.3, 122.6, 122.5, 120.9, 118.9, 116.8, 112.4, 111.7, 46.3, 27.1 ppm (2C). CHN: Berechnet für C₂₉H₂₃N: C 90.35, H 6.01, N 3.63; gefunden: C 89.98, H 6.31, N 3.49.

### Beispiel 4i: Synthese von Verbindung 3k:

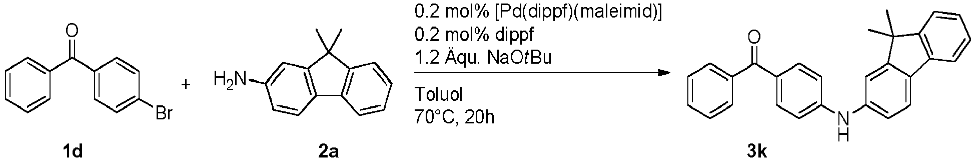

Nach der allgemeinen Vorschrift wurde Verbindung **3k** ausgehend von **1d** (269 mg, 1.00 mmol) und **2a** (209 mg, 1.00 mmol) dargestellt und konnte säulenchromatographisch (Al₂O₃, Diethylether:Hexan = 2:1) in 76% Ausbeute (296 mg, 0.76 mmol) isoliert werden. ¹H NMR (400 MHz, DMSO-*d₆*): δ = 9.08 (s, 1H), 7.80-7.45 (m, 10H), 7.41-7.35 (m, 1H), 7.33-7.13 (m, 5H), 1.43 ppm (s, 6H). ¹³C NMR (101 MHz, DMSO-*d₆*): *δ* = 193.6, 154.8, 152.9, 148.7, 140.6, 138.5 (2C), 132.9, 132.4 (2C), 131.5, 129.0 (2C), 128.3 (2C), 127.0, 126.7, 126.4, 122.6, 120.9, 119.3, 118.6, 114.1, 113.9 (2C), 46.4, 26.9 ppm (2C). CHN: Berechnet für C₂₈H₂₃NO: C 86.34, H 5.95, N 3.60; gefunden: C 86.05, H 6.10, N 3.52.

### Beispiele 5a bis 5h

### Allgemeine Vorschrift zur Herstellung tertiärer Amine

Alle Versuche wurden in 20 mL Headspace-Vials für die Gaschromatographie durchgeführt, die mit Aluminium-Bördelkappen mit Teflon-beschichteten Butylgummi-Septen verschlossen wurden (beides erhältlich z.B. bei der Firma *VWR*). Zur Temperierung der Gefäße wurden 8 cm hohe zylindrische Aluminiumblöcke verwendet, die in ihrem Durchmesser genau dem der Heizplatten von Labor-Magnetrührern (z.B. Heidolph Mr 2002) entsprechen. Diese Aluminiumblöcke wurden mit zehn 7 cm tiefen Bohrungen vom Durchmesser der Reaktionsgefäße und einer Bohrung zur Aufnahme eines Temperaturfühlers versehen.

Zum gleichzeitigen Evakuieren und Rückfüllen von jeweils zehn Gefäßen wurden Vakuumverteiler zum Anschluss an die Schlenk-Linie angefertigt. Dazu wurden jeweils zehn vakuumfeste 3 mm Teflonschläuche jeweils an einem Ende mit Adaptern zur Aufnahme von Luer-Lock-Spritzennadeln verbunden und mit dem anderen Ende an ein Stahlrohr angeschlossen, das über einen Vakuumschlauch mit der Schlenk-Linie verbunden werden kann.

Zur Durchführung der Versuche wurden das erste Arylbromid, X-B, (1.00 mmol), das entsprechende primäre Amin, A-NH₂, (1.00 mmol) und Natrium-*tert*-butanolat (118 mg, 1.20 mmol) an der Luft in die Reaktionsgefäße eingewogen, 20 mm Magnet-Rührkerne zugegeben und die Gefäße mittels einer Bördelzange mit Septumkappen luftdicht verschlossen. Jeweils 10 Reaktionsgefäße wurden in die Bohrungen eines Aluminiumblocks gesteckt und über Hohlnadeln, die durch die Septenkappen gebohrt wurden, mit dem Vakuumverteiler verbunden.

Die Reaktionsgefäße wurden danach gemeinsam dreimal hintereinander evakuiert und mit Stickstoff begast. Nachdem die Reaktionsgefäße auf diese Weise mit einer Inertgasatmosphäre versehen waren, wurde an der Vakuumlinie über ein Überdruckventil ein Druckausgleich mit der Außenatmosphäre hergestellt. Mit Hilfe einer Spritze wurde eine Stammlösung von [Pd(dippf)(maleimid)] (3.11 mg, 0.005 mmol) und dippf (2.13 mg, 0.005 mmol) in trockenem Toluol (2 mL) durch die Septenkappen hindurch injiziert. Danach wurde der Aluminiumblock auf 80 °C gebracht und die Nadeln des Vakuumverteilers entfernt.

Nach 20 h Reaktionszeit wurde das zweite Arylbromid, X-C, (1.10 mmol) in trockenem Toluol (0,5 mL) durch die Septenkappen hindurch injiziert. Die Temperatur wurde auf 120°C erhöht, das Reaktionsgemisch für 24 Stunden gerührt und nach Abkühlen auf Raumtemperatur vorsichtig geöffnet und das Reaktionsmedium mit Dichlormethan (30 mL) und Wasser (30 mL) verdünnt. Die wässrige Phase wurde von der organischen Phase getrennt und mit Dichlormethan (2 x 20 mL) extrahiert. Die vereinigten organischen Phasen wurden über Magnesiumsulfat getrocknet, filtriert und das Lösemittel im Vakuum entfernt (40°C, 500 mbar). Das verbliebene Rohprodukt wurde säulenchromatographisch (SiO₂, Ethylacetat: Hexan) gereinigt und so das entsprechende tertiäre Amin erhalten.

### Beispiel 5a: Herstellung von Verbindung 4b:

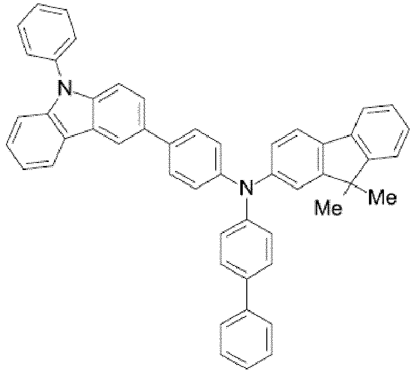

Verbindung 4b wurde nach der Allgemeinen Vorschrift erhalten, ausgehend von 3-(4-Bromphenyl)-9-phenyl-9H-carbazol (398 mg, 1 mmol), 9,9-Dimethyl-9H-fluoren-2-amin (209 mg, 1 mmol) und 4-Brom-1,1'-biphenyl (262 mg, 1.10 mmol) als zweites Arylhalogenid X-C. Durch Säulenchromatographie (Hexan:Ethylacetat 4:1) wurde die Verbindung als beigefarbener Feststoff erhalten (624 mg, 0,92 mmol, 92%). Schmelzpunkt: 155-156°C. ¹H NMR (400 MHz, DMSO-*d₆*) δ = 5.58 (d, *J*=1.5 Hz, 1H), 8.34 (d, *J*=7.8 Hz, 1H), 7.79 - 7.60 (m, 13H), 7.54 (m, 2H), 7.47 - 7.37 (m, 5H), 7.36 - 7.24 (m, 5H), 7.19 (d, *J*=8.8 Hz, 2H), 7.15 (d, *J*=8.5 Hz, 2H), 7.06 (dd, *J*=8.0 Hz, 2.0 Hz, 1H), 1.39 ppm (s, 6H); ¹³C NMR (101 MHz, DMSO-*d₆*) δ = 154.9, 153.2, 146.8, 146.4, 145.9, 140.6, 139.5, 139.4, 138.2, 136.8, 135.4, 134.1, 134.1, 132.0, 130.2 (2C), 128.9 (2C), 127.7 (2C), 127.7, 127.6 (2C), 127.1, 127.0, 126.7, 126.6 (2C), 126.4, 126.2 (2C), 124.9, 124.3, 123.6, 123.4, 123.4 (2C), 122.9, 122.7, 121.2, 120.8, 120.2, 119.6, 118.8, 118.2, 110.0, 109.7, 99.5, 46.5, 26.8 ppm (2C); IR v = 1599 (m), 1484 (s), 1474 (s), 1458 (s), 1449 (vs), 1298 (m), 1232 (s), 760 (s), 735 (vs), 696 cm-¹ (vs); CHN: berechnet (%) für C₅₁H₃₈N₂: C 90.23, H 5.64, N 4.13; gefunden: C 90.27, H 5.46, N 4.10.

### Beispiel 5b: Herstellung von Verbindung 4c:

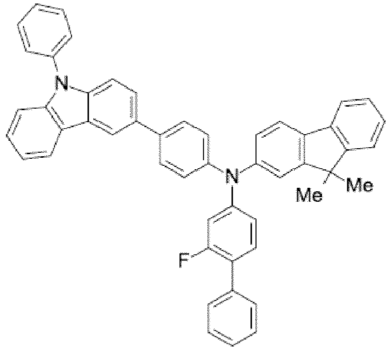

Verbindung 4c wurde nach der Allgemeinen Vorschrift erhalten, ausgehend von 3-(4-Bromphenyl)-9-phenyl-9H-carbazol (398 mg, 1 mmol), 9,9-Dimethyl-9H-fluoren-2-amin (209 mg, 1 mmol) und 4-Brom-2-fluor-1,1'-biphenyl (282 mg, 1.10 mmol) als zweites Arylhalogenid X-C. Durch Säulenchromatographie (Hexan:Ethylacetat 9:1) wurde die Verbindung als beigefarbener Feststoff erhalten (655 mg, 0,94 mmol, 94%). Schmelzpunkt: 171-172°C. ¹H NMR (400 MHz, DMSO-*d₆*) δ = 8.58 (d, *J*=1.5 Hz, 1H), 8.33 (d, *J*=7.8 Hz, 1H), 7.77 (m, 4H), 7.69 (m, 3H), 7.62 (m, 2H), 7.53 (m, 4H), 7.46 - 7.22 (m, 13H), 7.1 (dd, *J*=8.3 Hz, 2.0 Hz, 1H), 6.89 (dd, *J*=8.5 Hz, 2.3 Hz, 1H), 6.82 (dd, *J*=13.3 Hz, 2.3 Hz, 1H), 1.39 ppm (s, 6H); ¹³C NMR (101 MHz, DMSO-*d₆*) δ = 159.5 (d, *J*=245.8 Hz, 1C), 155.1, 153.3, 148.5 (d, *J*=10.3 Hz, 1C), 145.6, 145.1, 140.6, 139.5, 138.1, 136.8, 136.4, 135.0, 135.0 (d, *J*=1.5 Hz, 1C), 131.8, 131.2 (d, *J*=5.9 Hz, 1C), 130.2 (2C), 128.6 (2C), 128.4 (d, *J*=3.7 Hz, 2C), 127.9 (2C), 127.7, 127.3, 127.1, 127.0, 126.6 (2C), 126.5, 125.1 (2C), 125.0, 124.4, 123.4, 122.9, 122.7, 121.4, 121.0 (d, J=13.9 Hz, 1C), 120.8, 120.2, 119.8, 119.7, 118.3, 117.7 (d, *J*=1.5 Hz, 1C), 110.0, 109.7, 108.4 (d, *J*=25.7 Hz, 1C), 46.6, 26.7 ppm (2C); ¹⁹F NMR (376.5 MHz, DMSO-*d₆*) δ = -116.70 ppm (s, 1F); IR v = 1600 (m), 1501 (m), 1474 (s), 1458 (s), 1449 (s), 1310 (m), 1232 (m), 759 (s), 736 (s), 696 cm⁻¹ (vs); CHN: berechnet (%) für C₅₁H₃₇FN₂: C 87.9, H 5.35, N 4.02; gefunden: C 87.77, H 5.50, N 3.93.

### Beispiel 5c: Herstellung von Verbindung 4d:

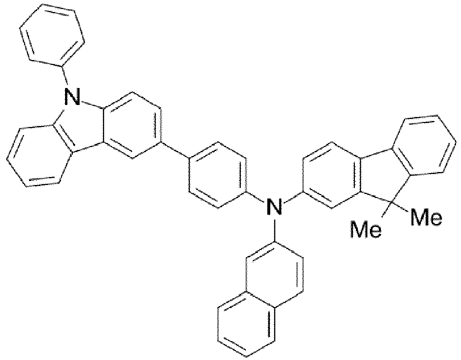

Verbindung 4c wurde nach der Allgemeinen Vorschrift erhalten, ausgehend von 3-(4-Bromphenyl)-9-phenyl-9H-carbazol (398 mg, 1 mmol), 9,9-Dimethyl-9H-fluoren-2-amin (209 mg, 1 mmol) und 2-Bromnaphthalin (230 mg, 1.10 mmol) als zweites Arylhalogenid X-C. Durch Säulenchromatographie (Hexan:Ethylacetat 9:1) wurde die Verbindung als beigefarbener Feststoff erhalten (628 mg, 0,96 mmol, 96%). Schmelzpunkt: 196-197°C. ¹H NMR (400 MHz, DMSO-*d₆*) δ = 8.58 (m, 1H), 8.33 (d, *J*=7.8 Hz, 1H), 7.84 (m, 2H), 7.77 - 7.60 (m, 10H), 7.57 - 7.47 (m, 3H), 7.46 - 7.35 (m, 5H), 7.34 (d, *J*=2.0 Hz, 1H), 7.33 - 7.23 (m, 4H), 7.19 (d, *J*=8.5 Hz, 2H), 7.05 (dd, *J*=8.3 Hz, 2.0 Hz, 1H), 1.36 ppm (s, 6H); ¹³C NMR (101 MHz, DMSO-*d₆*) δ = 154.9, 153.2, 146.6, 146.1, 145.0, 140.6, 139.4, 138.3, 136.8, 135.4, 134.1, 134.0, 131.9, 130.2 (2C), 129.7, 129.1, 127.7 (2C), 127.7, 127.5, 127.1, 126.9, 126.7, 126.6 (2C), 126.5, 126.4, 124.9, 124.6, 124.2 (2C), 124.0, 123.4, 123.3, 122.9, 122.7, 121.2, 120.8, 120.2, 119.6 (2C), 118.5, 118.2, 110.0, 109.7, 46.5, 26.7 ppm (2C); IR v = 1597 (m), 1501 (m), 1458 (s), 1449 (s), 1299 (m), 1229 (m), 807 (m), 745 (s), 735 (vs), 697 cm⁻¹ (m); CHN: berechnet (%) für C₄₉H₃₆N₂: C 90.15, H 5.56, N 4.29; gefunden: C 89.93, H 5.70, N 4.14.

### Beispiel 5d: Herstellung von Verbindung 4e:

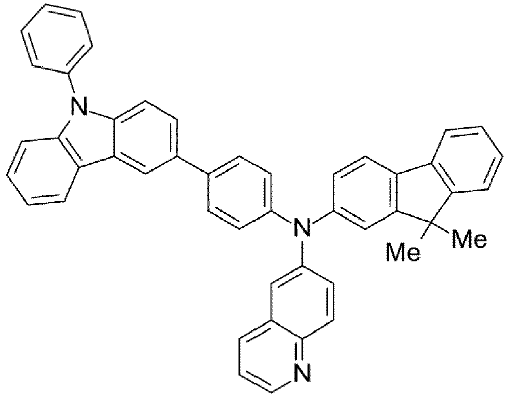

Verbindung 4e wurde nach der Allgemeinen Vorschrift erhalten, ausgehend von 3-(4-Bromphenyl)-9-phenyl-9H-carbazol (398 mg, 1 mmol), 9,9-Dimethyl-9H-fluoren-2-amin (209 mg, 1 mmol) und 6-Chlorchinolin (182 mg, 1.10 mmol) als zweites Arylhalogenid X-C. Durch Säulenchromatographie (Hexan:Ethylacetat 2:1) wurde die Verbindung als gelber Feststoff erhalten (595 mg, 0,91 mmol, 91%). Schmelzpunkt: 168-169°C. ¹H NMR (400 MHz, DMSO-*d₆*) δ = 8.74 (dd, *J*=4.3 Hz, 1.8 Hz, 1H), 8.58 (d, *J*=1.5 Hz, 1H), 8.33 (d, *J*=7.8 Hz, 1H), 8.14 (m, 1H), 7.93 (m, 1H), 7.78 - 7.60 (m, 9H), 7.56 - 7.47 (m, 4H), 7.45 - 7.35 (m, 5H), 7.34 - 7.20 (m, 5H), 7.08 (dd, *J*=8.3 Hz, 2.0 Hz, 1H), 1.37 ppm (s, 6H); ¹³C NMR (101 MHz, DMSO-*d₆*) δ = 155.0, 153.2, 148.7, 146.3, 145.7, 145.4, 144.6, 140.5, 139.4, 138.2, 136.8, 135.8, 134.7, 134.4, 131.8, 130.2 (2C), 130.1, 129.0, 127.8 (2C), 127.6, 127.1, 126.8, 126.8, 126.6 (2C), 126.4, 124.9, 124.5 (2C), 123.6, 123.4, 122.9, 122.7, 121.7, 121.2, 120.8, 120.1, 119.6, 118.9, 118.3, 118.2, 110.0, 109.7, 46.7, 26.7 ppm (2C); IR v = 1599 (m), 1497 (s), 1474 (s), 1458 (vs), 1448 (vs), 1299 (s), 1231 (vs), 758 (s), 735 (vs), 696 cm⁻¹ (s); CHN: berechnet (%) für C₄₈H₃₅N₃: C 88.18, H 5.40, N 6.43; gefunden: C 88.06, H 5.62, N 6.20.

### Beispiel 5e: Herstellung von Verbindung 4f:

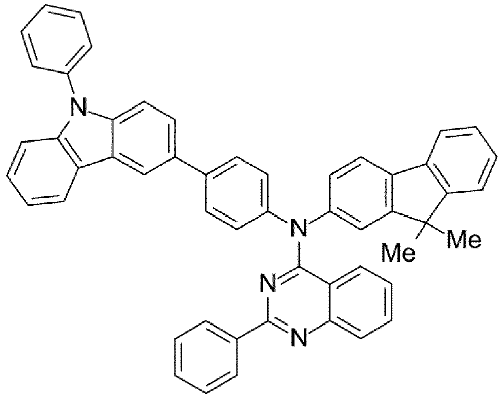

Verbindung 4f wurde nach der Allgemeinen Vorschrift erhalten, ausgehend von 3-(4-Bromphenyl)-9-phenyl-9H-carbazol (398 mg, 1 mmol), 9,9-Dimethyl-9H-fluoren-2-amin (209 mg, 1 mmol) und 4-Chlor-2-phenylchinazolin (273 mg, 1.10 mmol) als zweites Arylhalogenid X-C. Durch Säulenchromatographie (Hexan:Ethylacetat 4:1) wurde die Verbindung als gelber Feststoff erhalten (549 mg, 0,75 mmol, 75%). Schmelzpunkt: 206-207°C. ¹H NMR (400 MHz, DMSO-*d₆*) δ = 8.67 (d, *J*=1.5 Hz, 1H), 8.36 (d, *J*=7.8 Hz, 1H), 8.23 (dd, *J*=8.0 Hz, 1.8 Hz, 2H), 7.95 (d, *J*=8.0 Hz, 1H), 7.85 (dd, *J*=8.3 Hz, 5.3 Hz, 3H), 7.82 - 7.52 (m, 10H), 7.46 - 7.23 (m, 13H), 7.14 (dd, *J*=8.2 Hz, 1.9 Hz, 1H), 1.38 ppm (s, 6H); ¹³C NMR (101 MHz, DMSO-*d₆*) δ = 162.7, 158.7, 154.6, 153.5, 152.9, 145.9, 145.3, 140.6, 139.6, 138.0, 137.8, 137.7, 136.7, 135.9, 133.0, 131.4, 130.4, 130.2 (2C), 128.7, 128.3, 127.8 (2C), 127.7 (3C), 127.2, 127.1, 126.7 (2C), 126.6, 126.6 (2C), 126.5, 125.8, 125.5, 125.0, 124.9, 123.5, 122.9, 122.8, 121.2, 121.0, 120.9, 120.2, 120.0, 118.5, 116.4, 110.0, 109.7, 46.5, 26.6 ppm (2C); IR v = 1484 (vs), 1474 (vs), 1457 (s), 1375 (vs), 1331 (vs), 1232 (s), 759 (vs), 736 (vs), 707 (vs), 698 cm⁻¹ (vs); CHN: berechnet (%) für C₅₃H₃₈N₄: C 87.09, H 5.24, N 7.67; gefunden: C 86.78, H 5.48, N 7.55.

### Beispiel 5f: Herstellung von Verbindung 4g:

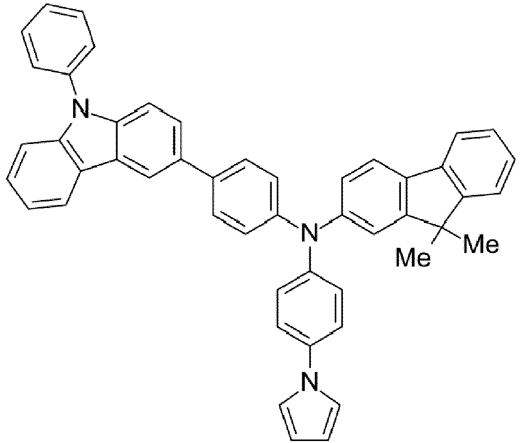

Verbindung 4g wurde nach der Allgemeinen Vorschrift erhalten, ausgehend von 3-(4-Bromphenyl)-9-phenyl-9H-carbazol (398 mg, 1 mmol), 9,9-Dimethyl-9H-fluoren-2-amin (209 mg, 1 mmol) und 1-(4-Chlorphenyl)-1H-pyrrol (199 mg, 1.10 mmol) als zweites Arylhalogenid X-C. Durch Säulenchromatographie (Hexan:Ethylacetat 9:1) wurde die Verbindung als farbloser Feststoff erhalten (633 mg, 0,95 mmol, 95%). Schmelzpunkt: 173-174°C. ¹H NMR (400 MHz, DMSO-*d₆*) δ = 8.57 (d, *J*=1.5 Hz, 1H), 8.34 (d, *J*=7.5 Hz, 1H), 7.77 - 7.62 (m, 9H), 7.57 - 7.48 (m, 4H), 7.47 - 7.37 (m, 3H), 7.34 - 7.24 (m, 6H), 7.16 (d, *J*=8.8 Hz, 4H), 7.03 (dd, *J*=8.2 Hz, 2.1 Hz, 1H), 6.25 (t, *J*=2.1 Hz, 2H), 1.39 ppm (s, 6H); ¹³C NMR (101 MHz, DMSO-*d₆*) δ = 154.9, 153.2, 146.6, 146.0, 144.6, 140.6, 139.4, 138.3, 136.8, 135.3, 135.1, 133.9, 132.0, 130.2 (2C), 127.7 (2C), 127.7, 127.1, 126.7, 126.6 (2C), 126.5, 124.9 (2C), 123.7 (2C), 123.4, 123.0, 122.9, 122.7, 121.2, 120.8, 120.6 (2C), 120.2, 119.6, 118.9 (2C), 118.2, 118.2 (2C), 110.3 (2C), 110.0, 109.7, 46.5, 26.8 ppm (2C); IR v = 1599 (w), 1512 (vs), 1501 (s), 1449 (s), 1311 (m), 1232 (m), 1069 (m), 735 (s), 723 (vs), 698 cm⁻¹ (s); CHN: berechnet (%) für C₄₉H₃₇N₃: C 88.12, H 5.58, N 6.29; gefunden: C 88.07, H 5.75, N 6.19.

### Beispiel 5g: Herstellung von Verbindung 4h:

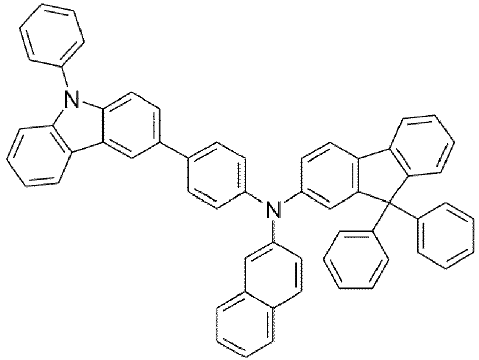

Verbindung 4h wurde nach der Allgemeinen Vorschrift erhalten, ausgehend von 3-(4-Bromphenyl)-9-phenyl-9H-carbazol (398 mg, 1 mmol), 9,9-Diphenyl-9H-fluoren-2-amin (333 mg, 1 mmol) und 2-Bromnaphthalin (230 mg, 1.10 mmol) als zweites Arylhalogenid X-C. Durch Säulenchromatographie (Hexan:Ethylacetat 9:1) wurde die Verbindung als beigefarbener Feststoff erhalten (743 mg, 0,96 mmol, 96%). Schmelzpunkt: 185-186°C. ¹H NMR (400 MHz, DMSO-*d₆*) δ = 8.48 (s, 1H), 8.27 (d, *J*=7.5 Hz, 1H), 7.77 - 7.65 (m, 4H), 7.61 - 7.47 (m, 8H), 7.47 - 7.40 (m, 2H), 7.38 - 7.20 (m, 8 H), 7.19 - 6.95 ppm (m, 16H); ¹³C NMR (101 MHz, DMSO-*d₆*) δ = 151.9, 150.3, 146.9, 145.5, 145.3 (2C), 144.6, 140.5, 139.4 (2C), 136.7, 135.8, 134.5, 133.9, 131.8, 130.1 (2C), 129.7, 129.7, 129.0, 128.2 (4C), 127.6 (br. s, 6C), 127.4, 127.0, 126.8, 126.6 (2C), 126.5 (3C), 126.4 (2C), 125.9, 124.8, 124.6, 124.5 (2C), 123.8, 123.4, 123.0, 122.9, 121.5, 121.1, 120.7, 120.1, 119.8, 119.6, 118.1, 109.9, 109.7, 64.8 ppm; IR v = 1596 (m), 1501 (m), 1474 (m), 1452 (s), 1295 (m), 1281 (m), 1231 (m), 805 (m), 743 (vs), 696 cm⁻¹(vs); CHN: berechnet (%) für C₅₉H₄₀N₂: C 91.21, H 5.19, N 3.61; gefunden: C 91.07, H 5.45, N 3.47.

### Beispiel 5h: Herstellung von Verbindung 4i:

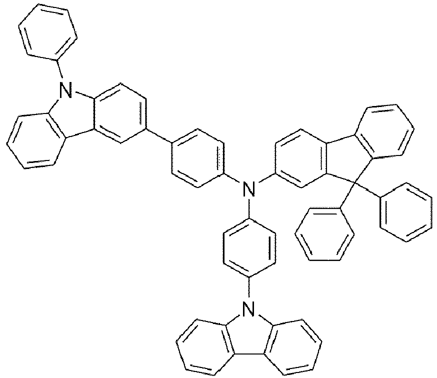

Verbindung 4i wurde nach der Allgemeinen Vorschrift erhalten, ausgehend von 3-(4-Bromphenyl)-9-phenyl-9H-carbazol (398 mg, 1 mmol), 9,9-Diphenyl-9H-fluoren-2-amin (333 mg, 1 mmol) und 9-(4-Bromphenyl)-9H-carbazol (414 mg, 1.10 mmol) als zweites Arylhalogenid X-C. Durch Säulenchromatographie (Hexan:Ethylacetat 4:1) wurde die Verbindung als beigefarbener Feststoff erhalten (849 mg, 0,95 mmol, 95%). Schmelzpunkt: 192-193°C. ¹H NMR (400 MHz, DMSO-*d₆*) δ = 8.45 (s, 1H), 8.23 (d, *J*=7.8 Hz, 1H), 8.16 (d, *J*=7.8 Hz, 2H), 7.76 - 7.53 (m, 9H), 7.48 - 7.35 (m, 12H), 7.34 - 7.14 ppm (m, 20H); ¹³C NMR (101 MHz, DMSO-*d₆*) δ = 153.8, 152.0, 148.3, 147.8, 146.9, 146.9 (2C), 142.2, 141.9 (2C), 141.0, 140.8, 138.7, 137.8, 136.2, 133.7, 132.6, 130.7 (2C), 129.1 (4C), 129.0 (4C), 129.0, 128.7 (2C), 128.4, 128.2, 128.0 (2C), 127.9, 127.5 (2C), 127.1, 127.0, 126.7 (2C), 125.8 (2C), 125.8 (2C), 125.3 (2C), 124.9, 124.6, 124.4, 124.1 (2C), 123.2, 122.0, 121.2, 121.1 (2C), 121.0, 120.6 (2C), 120.6, 119.1, 110.9, 110.7, 110.6 (2C), 66.4; IR v = 1598 (m), 1509 (s), 1475 (m), 1451 (vs), 1306 (m), 1286 (m), 1232 (m), 747 (vs), 724 (s), 699 cm⁻¹ (s); CHN: berechnet (%) für C₆₇H₄₅N₃: C 90.21, H 5.08, N 4.71; gefunden: C 89.99, H 5.18, N 4.72.

## Patentansprüche

1. Verfahren zur selektiven Arylierung eines primären aromatischen Amins der Formel A-NH₂ zu einem tertiären aromatischen Amin N-ABC, wobei die Reste A, B und C unabhängig voneinander gleiche oder verschiedene, substituierte oder unsubstituierte aromatische Reste sind, mindestens einer der Reste A, B oder C eine Biphenyleinheit aufweist, mit den Schritten
- Umsetzung des primären aromatischen Amins der Formel A-NH₂ mit einer aromatischen Verbindung der Formel X-B, wobei X ein Halogen oder ein Trifluormethylsulfonsäurerest ist, in Gegenwart eines ersten Katalysators bei einer ersten Reaktionstemperatur, um ein sekundäres Amin zu erhalten;
- Umsetzung des sekundären Amins mit einer aromatischen Verbindung der Formel X-C, wobei X ein Halogen oder ein Trifluormethylsulfonsäurerest ist, in Gegenwart eines zweiten Katalysators bei einer zweiten Reaktionstemperatur, um das tertiäre aromatische Amin N-ABC zu erhalten,
wobei die zweite Reaktionstemperatur höher ist als die erste Reaktionstemperatur, die Reaktionen in Gegenwart einer Base durchgeführt wird und der erste und der zweite Katalysator gleich oder verschieden und jeweils ein Palladiumkomplex sind, ausgewählt aus der Gruppe bestehend aus Bis-(Dialkylphosphinoferrocen)palladium-Komplexen, Komplexen der Formel PdX₁X₂L₁L₂ wobei X₁ und X₂ gleiche oder verschiedene Halogenliganden, L₁ und L₂ gleiche oder verschiedene neutrale Elektronendonorliganden sind, oder deren Kombinationen.

2. Verfahren nach Anspruch 1, wobei X1 und X2 gleich und ausgewählt aus F, Cl, Br oder I sind.

3. Verfahren nach Anspruch 1 oder 2, wobei L1 und L2 verschieden sind.

4. Verfahren nach einem oder mehreren der Ansprüche 1 bis 3, wobei L1 und L2 substituierte oder unsubstituierte neutrale aromatische oder heteroaromatische Verbindungen sind.

5. Verfahren nach einem oder mehreren der Ansprüche 1 bis 4, wobei L1 ein NHC-Ligand ist.

6. Verfahren nach einem oder mehreren der Ansprüche 1 bis 5, wobei L2 ein Pyridylligand ist.

7. Verfahren nach einem oder mehreren der Ansprüche 1 bis 6, wobei die Alkylsubstituenten der Bis-(Dialkylphosphinoferrocen)-Liganden zwei bis fünf Kohlenstoffatome aufweisen.

8. Verfahren nach Anspruch 1 oder 7, wobei die Alkylsubstituenten ausgewählt sind aus der Gruppe bestehend aus Isopropyl, Isobutyl, tert.-Butyl und deren Kombinationen.

9. Verfahren nach einem der Ansprüche 1 bis 8, wobei mindestens zwei der aromatischen Substituenten A, B oder C eine Biphenyleinheit aufweisen, welche gleich oder voneinander verschieden sein können.

10. Verfahren nach einem der Ansprüche 1 bis 9, wobei die Biphenyleinheit direkt an das sekundäre Stickstoffatom des Amins gebunden ist.

11. Verfahren nach einem der vorstehenden Ansprüche, wobei die Biphenyleinheit eine verbrückte Biphenyleinheit der Formel 2 oder 3 ist 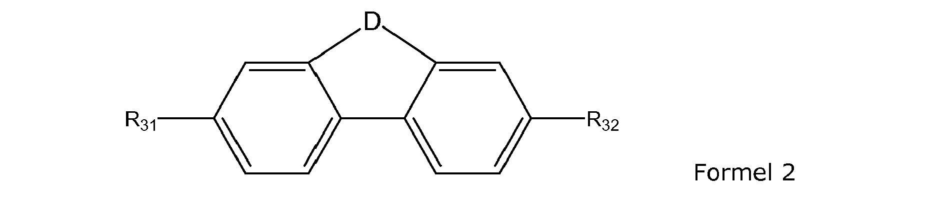 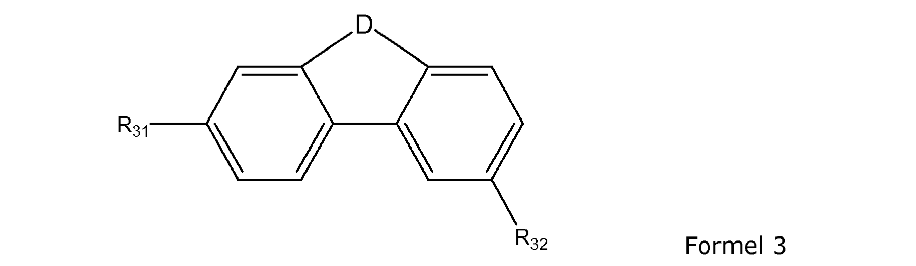 wobei D Sauerstoff, Schwefel, Stickstoff oder Kohlenstoff sein und im Fall von Stickstoff einfach oder im Fall von Kohlenstoff zweifach mit Methyl, Ethyl, Biphenyl, Naphthyl oder Phenyl substituiert sein kann, R31 Wasserstoff, Phenyl, Biphenyl oder Pyridyl sein kann, R32 eine Abgangsgruppe, Halogen, eine primäre Amingruppe NH₂ oder ein Trifluormethylsulfonsäurerest sein kann, je nachdem ob der Rest als A, B oder C verwendet wird; oder aber R32 ein Spacer ist, welcher zwischen A bzw. B oder C und X bzw. NH₂ angeordnet ist.

12. Verfahren nach einem oder mehreren der vorstehenden Ansprüche, wobei die Biphenyleinheit 2-Fluoren, 3-Fluoren, 2-(9,9-Diphenylfluoren), 2-(9,9-Dimethylfluoren), 3-(9,9-Diphenylfluoren), 3-(9,9-Dimethylfluoren), 3-(4-phenyl)-9-phenyl-9H-carbazol, 3-(4-phenyl)-9-methyl-9H-carbazol, 3-(4-phenyl)-9-Biphenyl-9H-carbazol, 2-(4-phenyl)-9-phenyl-9H-carbazol, 2-(4-phenyl)-9-methyl-9H-carbazol, 2-(4-phenyl)-9-Biphenyl-9H-carbazol, 3-(4-Biphenyl)-9-phenyl-9H-carbazol, 3-(4-Biphenyl)-9-methyl-9H-carbazol, 3-(4-Biphenyl)-9-Biphenyl-9H-carbazol, 2-(4-Biphenyl)-9-phenyl-9H-carbazol, 2-(4-Biphenyl)-9-methyl-9H-carbazol, 2-(4-Biphenyl)-9-Biphenyl-9H-carbazol, -- 3-(9-phenyl-9H-carbazol), 3-(9-methyl-9H-carbazol), 3-(9-Biphenyl-9H-carbazol), 2-(9-phenyl-9H-carbazol), 2-(9-methyl-9H-carbazol), 2-(9-Biphenyl-9H-carbazol) oder Triphenylene ist

13. Verfahren nach einem oder mehreren der vorstehenden Ansprüche, wobei der Palladiumkomplex als Feststoff, Lösung oder Pulvermischung verwendet wird.

14. Verfahren nach einem oder mehreren der vorstehenden Ansprüche, wobei in dem Palladiumkomplex das Palladiumatom zusätzlich von 2,4,6,8-Tetramethylcyclotetrasiloxan, Bis(dibenzylidenaceton) oder Maleimid komplexiert wird.

15. Verfahren nach einem oder mehreren der vorstehenden Ansprüche, wobei das Palladiumatom in dem Palladiumkomplex von 1,1'-Bis(diisopropylphosphino)ferrocen komplexiert wird.

16. Verfahren nach einem der vorstehenden Ansprüche enthaltend die Schritte:
- Bereitstellen des primären aromatischen Amins A-NH₂, des Halogenaromaten X-B, eines geeigneten Lösemittels und gegebenenfalls eines Bis-(Dialkylphosphinoferrocens) in einem Reaktionsgefäß;
- Zugeben eines Palladiumkomplexes, bei welchem das Palladiumatom von mindestens einem Bis-(Dialkylphosphinoferrocen)-Liganden komplexiert wird, in Form eines Feststoffs oder einer Lösung;
- Erwärmen des so erhaltenen Reaktionsgemisches in dem Reaktionsgefäß;
- Abtrennen des Reaktionsproduktes, eines sekundären aromatischen Amin A-NH-B; und
- Gegebenenfalls Reinigung des sekundären aromatischen Amin A-NH-B.

17. Verfahren nach einem der vorstehenden Ansprüche enthaltend die Schritte:
- Bereitstellen des primären aromatischen Amins A-NH₂, des Halogenaromaten X-B, eines geeigneten Lösemittels und gegebenenfalls eines Bis-(Dialkylphosphinoferrocens) in einem Reaktionsgefäß;
- Zugeben eines Palladiumkomplexes, bei welchem das Palladiumatom von mindestens einem Bis-(Dialkylphosphinoferrocen)-Liganden komplexiert wird, in Form eines Feststoffs oder einer Lösung;
- Erwärmen des so erhaltenen Reaktionsgemisches auf die erste Reaktionstemperatur;
- Zugeben eines Halogenaromaten X-C;
- Erwärmen auf die zweite Reaktionstemperatur; und
- Abtrennung und gegebenenfalls Reinigung des tertiären aromatischen Amin N-ABC.
